# EUROPEAN PATENT APPLICATION

(11) **EP 4 516 894 A1**
(43) Date of publication of application: **05.03.2025**
(21) Application number: 23796318.6
(22) Date of filing: 24.04.2023
(51) Int. Cl.: C12N 5/071, C12N 5/074

(54) **MATURING AGENT HAVING BOTH OF ALK5 INHIBITORY ACTIVITY AND CDK8/19 INHIBITORY ACTIVITY**

(30) Priority: 25.04.2022 JP 2022071507
(71) Applicant: Orizuru Therapeutics, Inc., Fujisawa-shi Kanagawa 251-8555 (JP)
(72) Inventor: SAKUMA Kensuke, Fujisawa-shi, Kanagawa 251-8555 (JP)
(74) Representative: Jones, Nicholas Andrew
(86) International application number: PCT/JP2023/016110
(87) International publication number: WO 2023/210578

(57) **Abstract**

An object of the present invention is to provide a novel method for inducing the differentiation of pluripotent stem cells into an insulin-positive cell population. The present invention provides a method for producing an insulin-positive cell population, comprising culturing and differentiating a pancreatic progenitor cell population or a cell population at a later stage of differentiation in a medium containing substantially no 2-(3-(6-methylpyridin-2-yl)-1H-pyrazol-4-yl)-1,5-naphthyridine and containing a factor having ALK5-inhibiting activity and CDK8/19-inhibiting activity.

## Description

### Technical Field

The present invention relates to a method for producing an insulin-positive cell population from pluripotent stem cells. More specifically, the present invention relates to a method for producing an insulin-positive cell population, comprising allowing a factor having inhibitory activity for cyclin-dependent kinase 8 and/or cyclin-dependent kinase 19 (hereinafter, also referred to as "CDK8/19") and inhibitory activity for ALK5 to act on a pancreatic progenitor cell population obtained by the induction of differentiation from pluripotent stem cells, or a cell population at a later stage of differentiation.

### [Background Art]

Research is underway to induce the differentiation of pluripotent stem cells such as iPS cells or ES cells into insulin-positive cells or pancreatic β cells and to apply the obtained cells to the treatment of diabetes mellitus.

Various approaches have been developed and reported so far in order to induce the differentiation of pluripotent stem cells into insulin-positive cell populations (Non Patent Literature 1). However, an insulin-positive cell population obtained by the induction of differentiation may comprise various cells such as residual relatively undifferentiated cells or malignantly transformed cells in addition to the insulin-positive cells (particularly, insulin-positive and NKX6.1-positive cells, etc.) of interest. In the case of applying an insulin-positive cell population to the treatment of diabetes mellitus, it is very important from a safety standpoint to strictly control the types of cells contained in the cell population. Hence, there has been a strong demand for a novel method for inducing differentiation into an insulin-positive cell population comprising a lower proportion of unintended cells.

### Citation List

### Non Patent Literature

Non Patent Literature 1: Stem Cell Research (2015) 14, 185-197

### Summary of Invention

### Technical Problem

The present inventors have found that in the process of inducing the differentiation of pluripotent stem cells into insulin-positive cell population, more specifically, in the process of inducing the differentiation of pancreatic progenitor cells into endocrine progenitor cells and/or cells at a later stage of differentiation, an ALK5 inhibitor 2-(3-(6-methylpyridin-2-yl)-1H-pyrazol-4-yl)-1,5-naphthyridine (also called "transforming growth factor-β type I receptor kinase", "activin receptor-like kinase 5 inhibitor II", "ALK5iII", "E 616452", "RepSox" or "SJN 2511"; hereinafter, also simply referred to as "ALK5iII" herein) which has heretofore been added to a medium for use might cause an irreversible change or a mutation in genetic information of organisms (that is, have mutagenicity). Although it is therefore preferred to avoid using ALK5iII in the process of inducing the differentiation, the present inventors have found that another ALK5 inhibitor used instead of ALK5iII reduces the induction efficiency of differentiation into the cell population of interest and cannot achieve the same production of the insulin-positive cell population as that by ALK5iII.

Accordingly, an object of the present invention is to find a novel differentiation-inducing factor that can efficiently perform the induction of differentiation mentioned above instead of ALK5iII and has low or no mutagenicity, and to provide a novel method for inducing the differentiation of pluripotent stem cells into an insulin-positive cell population using the factor.

### Solution to Problem

The present inventors have conducted diligent studies to attain the object and consequently found that in the process of inducing an insulin-positive cell population from pluripotent stem cells, a factor having ALK5-inhibiting activity and CDK8/19-inhibiting activity is allowed to act instead of ALK5iII on a pancreatic progenitor cell population or a cell population at a later stage of differentiation, whereby differentiation into insulin-positive cells, particularly, insulin-positive and NKX6.1-positive cells, can be efficiently induced and the induction of differentiation no way inferior to a conventional method using ALK5iII can be achieved.

The present invention is based on these novel findings and encompasses the following inventions.
[1] A method for producing an insulin-positive cell population, comprising
   culturing and differentiating a pancreatic progenitor cell population or a cell population at a later stage of differentiation in a medium containing a factor having ALK5-inhibiting activity and CDK8/19-inhibiting activity, wherein the medium contains substantially no 2-(3-(6-methylpyridin-2-yl)-1H-pyrazol-4-yl)-1,5-naphthyridine.
[2] The method according to [1], wherein the factor having ALK5-inhibiting activity and CDK8/19-inhibiting activity comprises an ALK5 inhibitor and a CDK8/19 inhibitor.
[3] The method according to [2], wherein the CDK8/19 inhibitor is one or more compounds selected from the group consisting of diethyl (E)-(4-(3-(5-(4-fluorophenyl)-1-methyl-1H-pyrazol-4-yl)acrylamido)benzyl)phosphonate, 2-(4-(4-(isoquinolin-4-yl)phenyl)-1H-pyrazol-1-yl)-N,N-dimethylacetamide, 4-((2-(6-(4-methylpiperazine-1-carbonyl)naphthalen-2-yl)ethyl)amino)quinazoline-6-carbonitrile, 4-(4-(2,3-dihydrobenzo[b][1,4]dioxin-6-yl)-1H-pyrazol-3-yl)benzene-1,3-diol, 3-(2-(imidazo[1,2-b]pyridazin-6-ylthio)ethyl)-4-(naphthalen-1-ylsulfornyl)-3,4-dihydroquinoxalin-2(1H)-one, and (E)-3-(4-(1-cyclopropyl-1H-pyrazol-4-yl)pyridin-3-yl)-N-(4-(morpholinomethyl)phenyl)acrylamide, or salt(s) thereof.
[4] The method according to [2], wherein the ALK5 inhibitor is one or more compounds selected from the group consisting of 4-[4-(1,3-benzodioxol-5-yl)-5-(2-pyridinyl)-1H-imidazol-2-yl]-benzamide, 6-[2-tert-butyl-5-(6-methylpyridin-2-yl)-1H-imidazol-4-yl]quinoxaline, 3-[[5-(6-methyl-2-pyridinyl)-4-(6-quinoxalinyl)-1H-imidazol-2-yl]methyl]benzamide, and 2-fluoro-N-[[5-(6-methylpyridin-2-yl)-4-([1,2,4]triazolo[1,5-a]pyridin-6-yl)-1H-imidazol-2-yl]methyl]aniline, or salt(s) thereof.
[5] The method according to [2], wherein the CDK8/19 inhibitor is 4-((2-(6-(4-methylpiperazine-1-carbonyl)naphthalen-2-yl)ethyl)amino)quinazoline-6-carbonitrile or 2-(4-(4-(isoquinolin-4-yl)phenyl)-1H-pyrazol-1-yl)-N,N-dimethylacetamide or a salt thereof, and the ALK5 inhibitor is 4-[4-(1,3-benzodioxol-5-yl)-5-(2-pyridinyl)-1H-imidazol-2-yl]-benzamide or a salt thereof.
[6] The method according to [1], wherein the pancreatic progenitor cell population or the cell population at a later stage of differentiation is a cell population produced by the induction of differentiation from pluripotent stem cells.
[7] A differentiation medium for a pancreatic progenitor cell population or a cell population at a later stage of differentiation, comprising a factor having ALK5-inhibiting activity and CDK8/19-inhibiting activity and containing substantially no 2-(3-(6-methylpyridin-2-yl)-1H-pyrazol-4-yl)-1,5-naphthyridine.
[8] The medium according to [7], wherein the factor having ALK5-inhibiting activity and CDK8/19-inhibiting activity comprises an ALK5 inhibitor and a CDK8/19 inhibitor.
[9] The medium according to [8], wherein the CDK8/19 inhibitor is one or more compounds selected from the group consisting of diethyl (E)-(4-(3-(5-(4-fluorophenyl)-1-methyl-1H-pyrazol-4-yl)acrylamido)benzyl)phosphonate, 2-(4-(4-(isoquinolin-4-yl)phenyl)-1H-pyrazol-1-yl)-N,N-dimethylacetamide, 4-((2-(6-(4-methylpiperazine-1-carbonyl)naphthalen-2-yl)ethyl)amino)quinazoline-6-carbonitrile, 4-(4-(2,3-dihydrobenzo[b][1,4]dioxin-6-yl)-1H-pyrazol-3-yl)benzene-1,3-diol, 3-(2-(imidazo[1,2-b]pyridazin-6-ylthio)ethyl)-4-(naphthalen-1-ylsulfornyl)-3,4-dihydroquinoxalin-2(1H)-one, and (E)-3-(4-(1-cyclopropyl-1H-pyrazol-4-yl)pyridin-3-yl)-N-(4-(morpholinomethyl)phenyl)acrylamide, or salt(s) thereof.
[10] The medium according to [8], wherein the ALK5 inhibitor is one or more compounds selected from the group consisting of 4-[4-(1,3-benzodioxol-5-yl)-5-(2-pyridinyl)-1H-imidazol-2-yl]-benzamide, 6-[2-tert-butyl-5-(6-methylpyridin-2-yl)-1H-imidazol-4-yl]quinoxaline, 3-[[5-(6-methyl-2-pyridinyl)-4-(6-quinoxalinyl)-1H-imidazol-2-yl]methyl]benzamide, and 2-fluoro-N-[[5-(6-methylpyridin-2-yl)-4-([1,2,4]triazolo[1,5-a]pyridin-6-yl)-1H-imidazol-2-yl]methyl]aniline, or salt(s) thereof.
[11] The medium according to [8], wherein the CDK8/19 inhibitor is 4-((2-(6-(4-methylpiperazine-1-carbonyl)naphthalen-2-yl)ethyl)amino)quinazoline-6-carbonitrile or 2-(4-(4-(isoquinolin-4-yl)phenyl)-1H-pyrazol-1-yl)-N,N-dimethylacetamide or a salt thereof, and the ALK5 inhibitor is 4-[4-(1,3-benzodioxol-5-yl)-5-(2-pyridinyl)-1H-imidazol-2-yl]-benzamide or a salt thereof.
[12] The medium according to [7], wherein the pancreatic progenitor cell population or the cell population at a later stage of differentiation is a cell population produced by the induction of differentiation from pluripotent stem cells.

The present specification encompasses the contents described in the specification, etc. of Japanese Patent Application No. 2022-071507 filed on April 25, 2022 on which the priority of the present application is based.

All publications, patents and patent applications cited herein are incorporated herein by reference in their entirety.

### Advantageous Effects of Invention

The present invention can provide a novel differentiation-inducing factor that can be used instead of ALK5iII, has low or no mutagenicity, and is capable of efficiently performing the induction of differentiation of pluripotent stem cells into an insulin-positive cell population, and can provide a novel method for inducing the differentiation of pluripotent stem cells into an insulin-positive cell population using the factor.

### Brief Description of Drawings

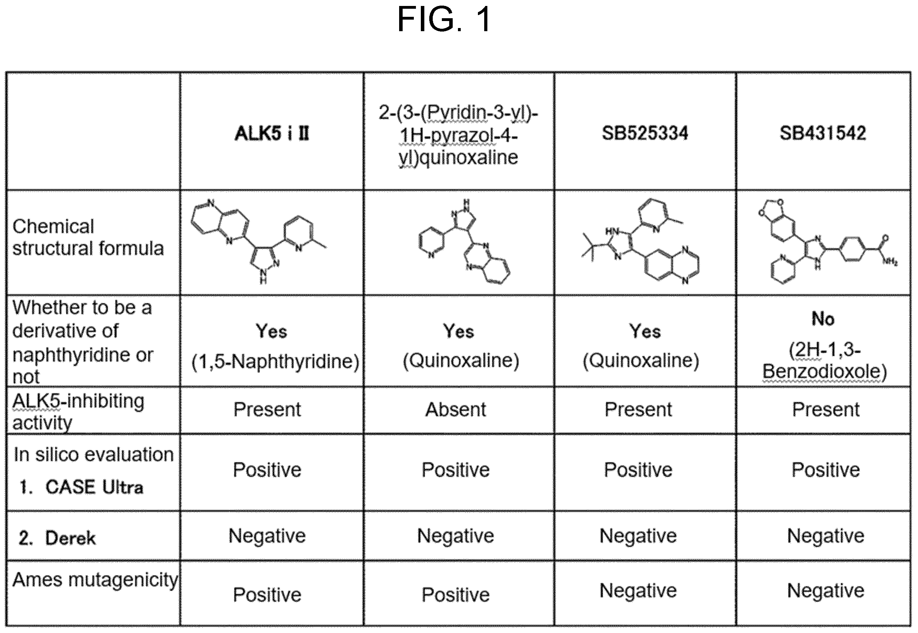

[Figure 1] Figure 1 shows a chemical structural formula, ALK5-inhibiting activity, and various evaluation results of mutagenicity as to ALK5iII, a structural analog of ALK5iII (2-(3-(pyridin-3-yl)-1H-pyrazol-4-yl)quinoxaline), and other representative ALK5 inhibitors (SB525334 and SB431542).

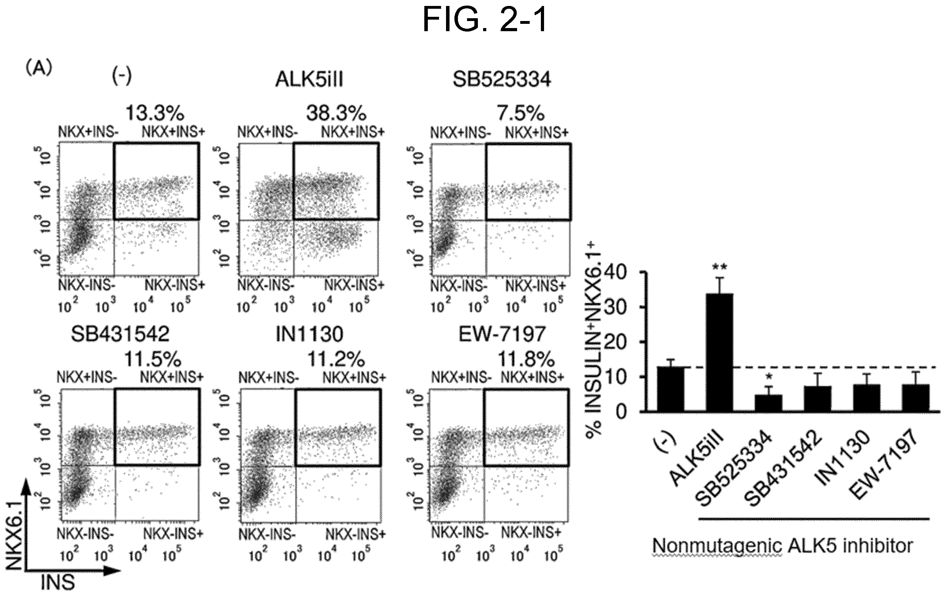

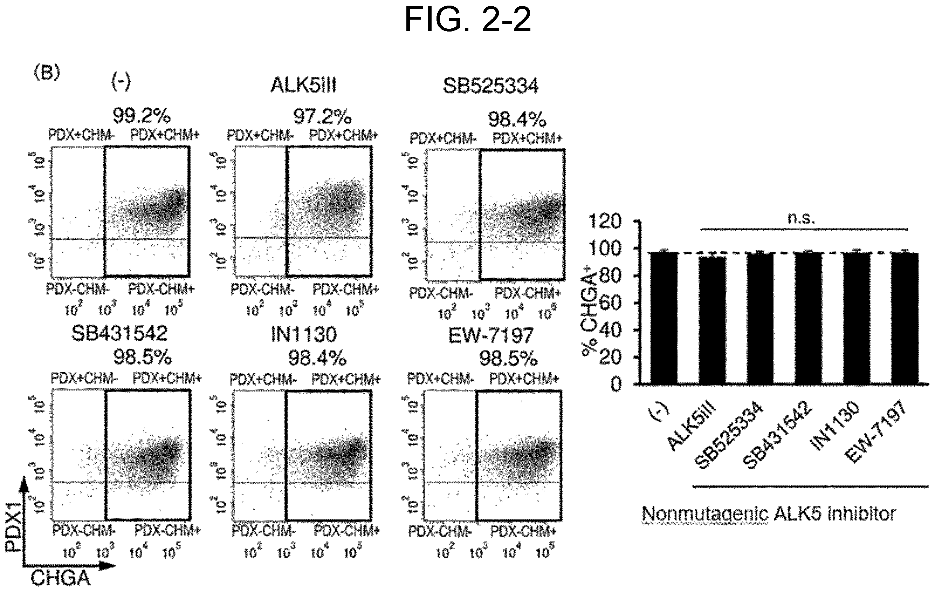

[Figure 2] Figure 2 shows flow cytometry results and a graph diagram showing each of (A) the proportion of insulin-positive and NKX6.1-positive (INSULIN⁺NKX6.1⁺) cells and (B) the proportion of CHGA-positive (CHGA⁺) cells in an insulin-positive cell population obtained by treating a pancreatic progenitor cell population with a medium for induction of differentiation containing ALK5 inhibitor II or a nonmutagenic ALK5 inhibitor (SB525334, SB431542, IN1130, or EW-7197).

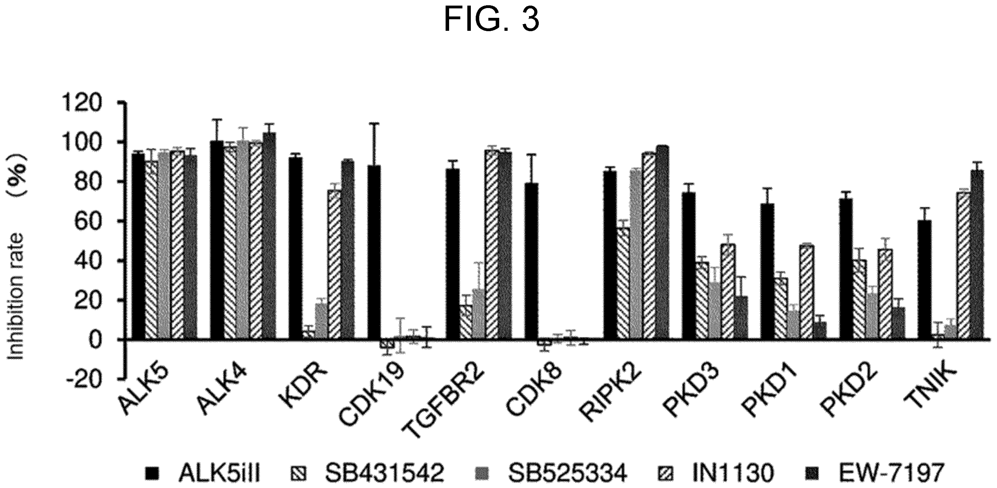

[Figure 3] Figure 3 is a graph diagram showing results of analyzing the inhibitory activity of ALK5iII and nonmutagenic ALK5 inhibitors (SB525334, SB431542, IN1130, and EW-7197) against 11 kinases. The results are indicated in relative values when the complete inhibition of the binding between each kinase and a tracer by an inhibitor is defined as 100% in a binding evaluation test of the kinase inhibitors.

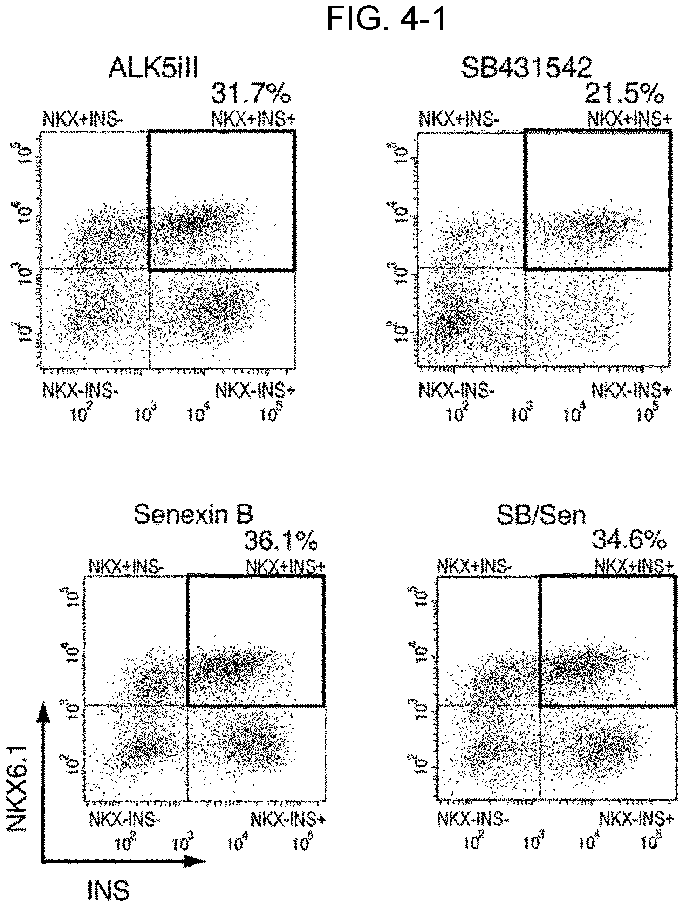

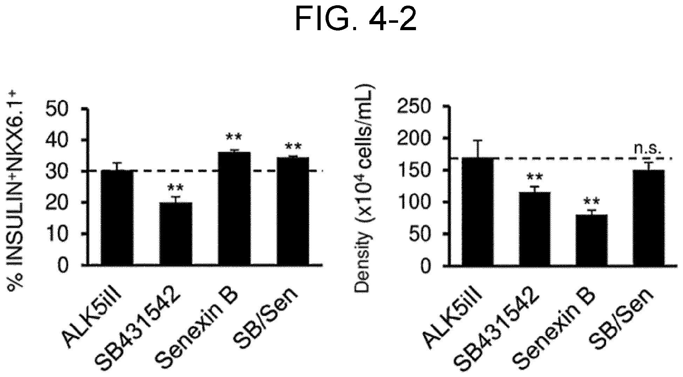

[Figure 4] Figure 4 shows flow cytometry results and a graph diagram showing (A) the proportion of insulin-positive and NKX6.1-positive (INSULIN⁺NKX6.1⁺) cells in each insulin-positive cell population obtained by treating a pancreatic progenitor cell population with a medium for induction of differentiation containing (1) ALK5iII, (2) a nonmutagenic ALK5 inhibitor (SB431542), (3) a CDK8/19 inhibitor (Senexin B), or (4) a combination of SB431542 and Senexin B (SB/Sen), and a graph diagram of (B) a total cell yield.

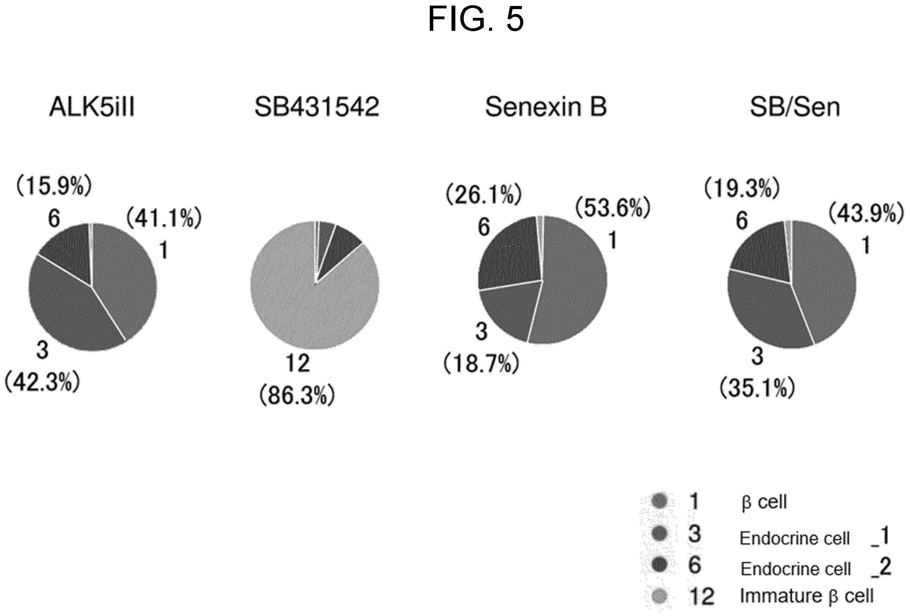

[Figure 5] Figure 5 is a pie chart showing the percentages of clusters #1, #3, #6, and #12 in an insulin-positive and NKX6.1-positive cell subpopulation in each insulin-positive cell population obtained by treating a pancreatic progenitor cell population with a medium for induction of differentiation containing (1) ALK5iII, (2) a nonmutagenic ALK5 inhibitor (SB431542), (3) a CDK8/19 inhibitor (Senexin B), or (4) a combination of SB431542 and Senexin B (SB/Sen).

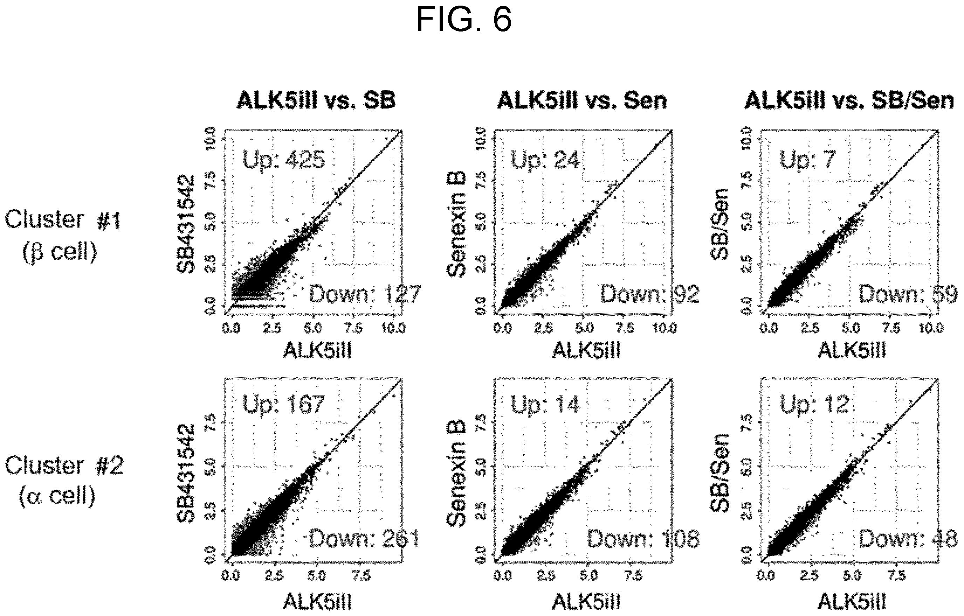

[Figure 6] Figure 6 shows results of analyzing a differentially expressed gene (DEG) between two samples as to clusters #1 and #2 in each insulin-positive cell population obtained by treating a pancreatic progenitor cell population with a medium for induction of differentiation containing (1) ALK5iII, (2) a nonmutagenic ALK5 inhibitor (SB431542), (3) a CDK8/19 inhibitor (Senexin B), or (4) a combination of SB431542 and Senexin B (SB/Sen).

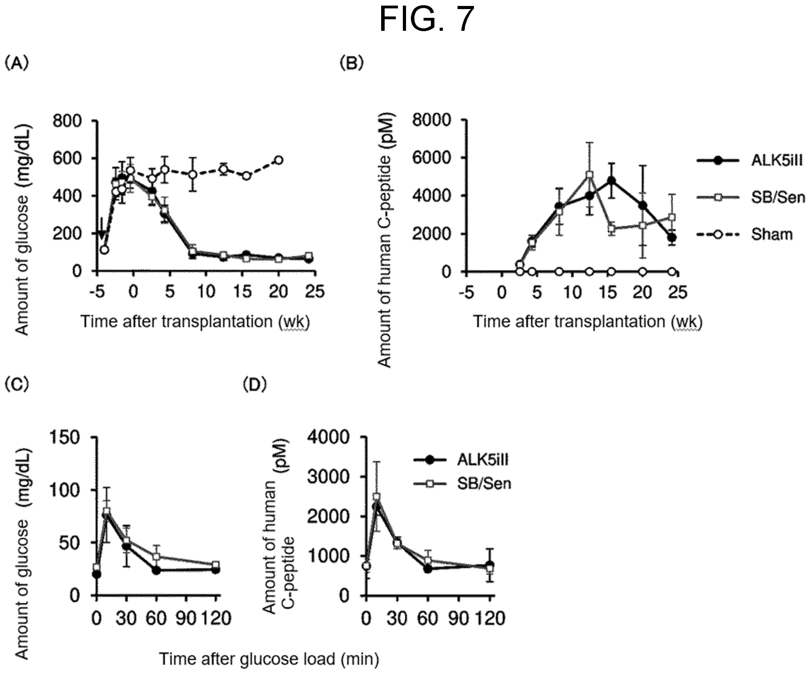

[Figure 7] Figure 7 is a graph diagram showing results of measuring (A) the amount of glucose and (B) the amount of human C-peptide in plasma in type I diabetes mellitus mouse models given a transplant of an insulin-positive cell population obtained by treating a pancreatic progenitor cell population with a medium for induction of differentiation containing (1) ALK5iII or (4) a combination of SB431542 and Senexin B (SB/Sen), and results of measuring (C) the amount of glucose and (D) the amount of human C-peptide in plasma in an oral glucose load test of the mouse models. Type I diabetes mellitus mouse models of a sham operation group (Sham) were used as controls.

### Description of Embodiments

### 1. Terminology

Hereinafter, the terms described herein will be described.

As used herein, "about" or "around" refers to a value which may vary up to plus or minus 25%, 20%, 10%, 8%, 6%, 5%, 4%, 3%, 2%, or 1% from the reference value. Preferably, the term "about" or "around" refers to a range from minus or plus 15%, 10%, 5%, or 1% from the reference value.

As used herein, "comprise(s)" or "comprising" means inclusion of the element(s) following the word without limitation thereto. Accordingly, it indicates inclusion of the element(s) following the word, but does not indicate exclusion of any other element.

As used herein, "consist(s) of" or "consisting of" means inclusion of all the element(s) following the phrase and limitation thereto. Accordingly, the phrase "consist(s) of" or "consisting of" indicates that the enumerated element(s) is required or essential and substantially no other elements exist.

As used herein, "without the use of feeder cell(s)" means basically containing no feeder cells and using no medium preconditioned by culturing feeder cells. Accordingly, the medium does not contain any substance, such as a growth factor or a cytokine, secreted by feeder cells.

"Feeder cells" or "feeder" means cells that are co-cultured with another kind of cells, support the cells, and provide an environment that allows the cells to grow. The feeder cells may be derived from the same species as or a different species from the cells that they support. For example, as a feeder for human cells, human skin fibroblasts or human embryonic-stem cells may be used or a primary culture of murine embryonic fibroblasts or immortalized murine embryonic fibroblasts may be used. The feeder cells can be inactivated by exposure to radiation or treatment with mitomycin C.

As used herein, "adhered (adherent)" refers to cells are attached to a container, for example, cells are attached to a cell culture dish or a flask made of a sterilized plastic (or coated plastic) in the presence of an appropriate medium. Some cells cannot be maintained or grow in culture without adhering to the cell culture container. In contrast, non-adherent cells can be maintained and proliferate in culture without adhering to the container.

As used herein, "culture" refers to maintaining, growing, and/or differentiating cells in in vitro environment. "Culturing" means maintaining, proliferating, and/or differentiating cells out of tissue or the living body, for example, in a cell culture dish or flask. The culture includes two-dimensional culture (plane culture) and three-dimensional culture (suspension culture).

As used herein, "enrich(es)" and "enrichment" refer to increasing the amount of a certain component in a composition such as a composition of cells and "enriched" refers, when used to describe a composition of cells, for example, a cell population, to a cell population increased in the amount of a certain component in comparison with the percentage of such component in the cell population before the enrichment. For example, a composition such as a cell population can be enriched for a target cell type and, accordingly, the percentage of the target cell type is increased in comparison with the percentage of the target cells present in the cell population before the enrichment. A cell population can be enriched for a target cell type by a method of selecting and sorting cells known in the art. A cell population can be enriched by a specific process of sorting or selection described herein. In a certain embodiment of the present invention, a cell population is enriched for a target cell population at least 20%, 30%, 40%, 50%, 60%, 70%, 80%, 85%, 90%, 95%, 97%, 98%, or 99% by a method of enriching the target cell population.

As used herein, "deplete(s)" and "depletion" refer to decreasing the amount of a certain component in a composition such as a composition of cells and "depleted" refers, when used to describe a composition of cells, for example, a cell population, to a cell population decreased in the amount of a certain component in comparison with the percentage of such component in the cell population before the depletion. For example, a composition such as a cell population can be depleted for a target cell type and, accordingly, the percentage of the target cell type is decreased in comparison with the percentage of the target cells present in the cell population before the depletion. A cell population can be depleted for a target cell type by a method of selecting and sorting cells known in the art. A cell population can be depleted by a specific process of sorting or selection described herein. In a certain embodiment of the present invention, a cell population is reduced (depleted) for a target cell population at least 50%, 80%, 85%, 90%, 95%, 97%, 98%, or 99% by a method of depleting a target cell population.

As used herein, "purify(ies)" and "purification" refer to removing impurities in a composition such as a composition of cells and making it pure for a certain component and "purified" refers, when used to describe a composition of cells, for example, a cell population, to a cell population in which the amount of impurities is decreased in comparison with the percentage of such components in the cell population before purification and the purity of a certain component is improved. For example, a composition such as a cell population can be purified for a target cell type and, accordingly, the percentage of the target cell type is increased in comparison with the percentage of the target cells present in the cell population before the purification. A cell population can be purified for a target cell type by a method of selecting and sorting cells known in the art. A cell population can be purified by a specific process of sorting or selection described herein. In a certain embodiment of the present invention, the purity of a target cell population is brought by a method of purifying a target cell population to at least 70%, 80%, 85%, 90%, 95%, 97%, 98%, or 99% or to the extent at which impurities (including contaminant cells) are undetectable.

As used herein, "marker" means a cell antigen or a gene thereof that is specifically expressed depending on a predetermined cell type, such as "marker protein" and "marker gene". Preferably, a marker is a cell surface marker and this allows concentration, isolation, and/or detection of living cells. A marker can be a positive selection marker or a negative selection marker.

The detection of a marker protein can be conducted by an immunological assay, for example, ELISA, immunostaining, or flow cytometry using an antibody specific for the marker protein. The detection of a marker gene can be conducted by a method of amplifying and/or detecting nucleic acid known in the art, for example, RT-PCR, microarray, biochip, or the like. As used herein, "positive" for a marker protein means being detected to be positive by flow cytometry and "negative" therefor means being equal to or less than the lower detection limit in flow cytometry. Also, as used herein, "positive" for a marker gene means being detected by RT-PCR and "negative" therefor means being equal to or less than the lower detection limit in RT-PCR.

As used herein, "expression" is defined as transcription and/or translation of a certain nucleotide sequence driven by an intracellular promoter.

As used herein, "growth factors" are endogenous proteins that promote differentiation and/or proliferation of particular cells. Examples of "growth factors" include epidermal growth factor (EGF), acid fibroblast growth factor (aFGF), basic fibroblast growth factor (bFGF), hepatocyte growth factor (HGF), insulin-like growth factor 1 (IGF-1), insulin-like growth factor 2 (IGF-2), keratinocyte growth factor (KGF), nerve growth factor (NGF), platelet-derived growth factor (PDGF), transformation growth factor beta (TGF-β), vascular endothelial growth factor (VEGF), transferrin, various interleukins (for example, IL-1 to IL-18), various colony stimulating factors (for example, granulocyte/macrophage-colony stimulating factor (GM-CSF)), various interferons (for example, IFN-y, and the like), and other cytokines having effects on stem cells, for example, stem cell factor (SCF), and erythropoietin (Epo).

As used herein, "ROCK inhibitors" means substances that inhibit Rho kinase (ROCK: Rho-associated, coiled-coil containing protein kinase) and may be substances that inhibit either of ROCK I and ROCK II. The ROCK inhibitors are not particularly limited as long as they have the aforementioned function and examples include N-(4-pyridinyl)-4β-[(R)-1-aminoethyl]cyclohexane-1α-carboxamide (that may be herein also referred to as Y-27632), Fasudil (HA1077), (2S)-2-methyl-1-[(4-methyl-5-isoquinolinyl]sulfonyl]hexahydro-1H-1,4-diazepine (H-1152), 4β-[(1R)-1-aminoethy1]-N-(4-pyridyl)benzene-1α-carbamide (Wf-536), N-(1H-pyrrolo[2,3-b]pyridin-4-yl)-4PER(R)-1-aminoethyl]cyclohexane-1α-carboxamide (Y-30141), N-(3-{[2-(4-amino-1,2,5-oxadiazol-3-yl)-1-ethyl-1H-imidazo[4,5-c]pyridin-6-yl]oxy}phenyl)-4-{[2-(4-morpholinyl)ethyl]-oxy}benzamide (GSK269962A), N-(6-fluoro-1H-indazol-5-yl)-6-methyl-2-oxo-4-[4-(trifluoromethyl)phenyl]-3,4-dihydro-1H-pyridine-5-carboxamide (GSK429286A). The ROCK inhibitors are not limited to these and antisense oligonucleotides and siRNA to ROCK mRNA, antibodies that bind to ROCK, and dominant negative ROCK mutants can also be used, commercially available, or synthesized according to a known method as ROCK inhibitors.

As used herein, "GSK3β inhibitors" are substances having the inhibitory activity for GSK3β (glycogen synthase kinase 3β). GSK3 (glycogen synthase kinase 3) is a serine/threonine protein kinase and involved in many signaling pathways associated with the production of glycogen, apoptosis, maintenance of stem cells, etc. GSK3 has the 2 isoforms α and β. "GSK3β inhibitors" used in the present invention are not particularly limited as long as they have the GSK3β-inhibiting activity and they may be substances having both the GSK3α-inhibiting activity and the GSK3β-inhibiting activity.

Examples of GSK3β inhibitors include CHIR98014 (2-[[2-[(5-nitro-6-aminopyridin-2-yl)amino]ethyl]amino]-4-(2,4-dichlorophenyl)-5-(1H-imidazol-1-yl)pyrimidine), CHIR99021 (6-[[2-[[4-(2,4-dichlorophenyl)-5-(4-methyl-1H-imidazol-2-yl)-2-pyrimidinyl]amino]ethyl]amino]nicotinonitrile), TDZD-8 (4-benzyl-2-methyl-1,2,4-thiadiazolidine-3,5-dione), SB216763 (3-(2,4-dichlorophenyl)-4-(1-methyl-1H-indol-3-yl)-1H-pyrrole-2,5-dione), TWS-119 (3-[6-(3-aminophenyl)-7H-pyrrolo[2,3-d]pyrimidin-4-yloxy]phenol), kenpaullone, 1-azakenpaullone, SB216763 (3-(2,4-dichlorophenyl)-4-(1-methyl-1H-indol-3-yl)-1H-pyrrole-2,5-dione), SB415286 (3-[(3-chloro-4-hydroxyphenyl)amino]-4-(2-nitrophenyl)-1H-pyrrole-2,5-dione), and AR-AO144-18, CT99021, CT20026, BIO, BIO-acetoxime, pyridocarbazole-ruthenium cyclopentadienyl complex, OTDZT, alpha-4-dibromoacetophenone, lithium, and the like. GSK3β is not limited to these and antisense oligonucleotides and siRNA to GSK3β mRNA, antibodies that bind to GSK3β, dominant negative GSK3β mutants, and the like can also be used, commercially available, or synthesized according to a known method as GSK3β inhibitors.

As used herein, examples of "serum replacement" include KnockOut(TM) Serum Replacement (KSR: Thermo Fisher Scientific), StemSure(R) Serum Replacement (Wako), B-27 supplement, N2-supplement, albumin (for example, lipid rich albumin), insulin, transferrin, fatty acids, collagen precursors, trace elements (for example, zinc, selenium (for example, sodium selenite)), 2-mercaptoethanol, 3'-thiolglycerol, or mixtures thereof (for example, ITS-G). Preferred serum replacements are B-27 supplement, KSR, StemSure(R) Serum Replacement, ITS-G. The concentration of serum replacement in a medium when added into a medium is 0.01-10% by weight, and preferably 0.1-2% by weight. In the present invention, "serum replacement" is preferably used instead of serum.

### 2. Method for producing insulin-positive cell population

The present invention provides a method for producing an insulin-positive cell population, comprising culturing and differentiating a pancreatic progenitor cell population or a cell population at a later stage of differentiation in a medium containing a factor having ALK5-inhibiting activity and CDK8/19-inhibiting activity and containing substantially no ALK5iII.

In the present invention, the medium can contain a factor having ALK5-inhibiting activity and CDK8/19-inhibiting activity. The factor may be one (or single) inhibitor having both ALK5-inhibiting activity and CDK8/19-inhibiting activity. Alternatively, the factor may be a combination of a plurality of factors consisting of a factor having ALK5-inhibiting activity (ALK5 inhibitor) and a factor having CDK8/19-inhibiting activity (CDK8/19 inhibitor). Preferably, in the present invention, the medium contains a combination of an ALK5 inhibitor and a CDK8/19 inhibitor.

"Contain substantially no ALK5iII" described about the medium of the present invention means that the medium does not contain ALK5iII in a form that exerts arbitrary activity including ALK5-inhibiting activity, and does not necessarily mean that the medium does not contain any ALK5iII. Preferably, this phrase means that the medium of the present invention does not contain any ALK5iII. In the present invention, "ALK5iII" includes salt and solvate forms of ALK5iII as long as these forms have mutagenicity.

As used herein, "factor having CDK8/19-inhibiting activity" or "CDK8/19 inhibitor" means any substance having the inhibitory activity for CDK8/19. CDK8, in contrast to the other proteins of the same CDK family, is not required for cell proliferation. The inhibition of CDK8 has no great effect under usual conditions. CDK19 and CDK8 are similar to each other. Usually, the inhibition of CDK8 also involves the inhibition of CDK19.

"CDK8/19 inhibitor" used in the present invention is not particularly limited as long as the CDK8/19 inhibitor has CDK8/19-inhibiting activity. Any factor that directly or indirectly inhibits the function of CDK8/19 can be used.

In the present invention, conventionally known "CDK8/19 inhibitor" can be used, and such a CDK8/19 inhibitor can be found from patent literatures or non patent literatures. For example, among compounds described in US2012/0071477, WO2015/159937, WO2015/159938, WO2013/116786, WO2014/0038958, WO2014/134169, JP2015/506376, US2015/0274726, US2016/0000787, WO2016/009076, WO2016/0016951, WO2016/018511, WO2016/100782 and WO2016/182904, a compound or a salt thereof having CDK8/19-inhibiting activity can be used as "CDK8/19 inhibitor" according to the present invention. Among the compounds described above, particularly, a compound or a salt thereof having activity of selectively inhibiting CDK8/19 can be suitably used.

More specifically, examples of the CDK8/19 inhibitor that may be used in the present invention include, but are not particularly limited to, compounds given below and salts thereof. These compounds may have one or more substituents or their substructures (substituents, rings, etc.) may be partially converted as long as the compounds have CDK8/19-inhibiting activity.

**[Table 1]**

| Compound | IUPAC Name | Structural formula |
|---|---|---|
| 1 | Diethyl (E)-(4-(3-(5-(4-fluorophenyl)-1-methyl-1H-pyrazol-4-yl)acrylamido)benzyl)phosphonate | 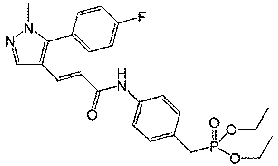 |
| 2 | 2-(4-(4-(lsoquinolin-4-yl)phenyl)-1H-pyrazol-1-yl)-N,N-dimethylacetamide | 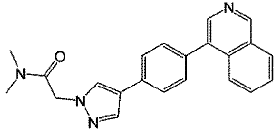 |
| 3 | 4-((2-(6-(4-Methylpiperazine-1-carbonyl)naphthalen-2-yl)ethyl)amino)quinazoline-6-carbonitrile | 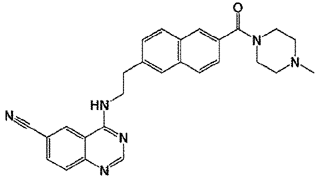 |
| 4 | 4-(4-(2,3-Dihydrobenzo[b][1,4]dioxin-6-yl)-1H-pyrazol-3-yl)benzene-1,3-diol | 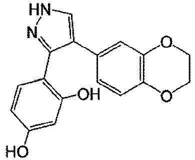 |
| 5 | 3-(2-(lmidazo[1,2-b]pyridazin-6-ylthio)ethyl)-4-(naphthalen-1-ylsulfornyl)-3,4-dihydroquinoxalin-2(1H)-one | 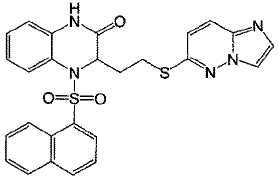 |
| 6 | (E)-3-(4-(1-Cyclopropyl-1H-pyrazol-4-yl)pyridin-3-yl)-N-(4-(morpholinomethyl)phenyl)acrylamide | 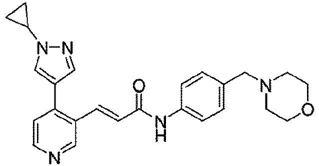 |

Examples of the CDK8/19 inhibitor that may be used in the present invention can include, but are not particularly limited to, compounds 7 to 13 described below and salts thereof. Each of compounds 7 to 11 is preferably in a free form, and each of compounds 12 and 13 is preferably trifluoroacetate.

**[Table 2]**

| Compound | **IUPAC** Name | Structural formula | Salt | **MS** |
|---|---|---|---|---|
| 7 | 8-(2,4-Difluorophenoxy)-1-methyl-4,5-dihydro-1H-thieno[3,4-g]indazole-6-carboxamide | 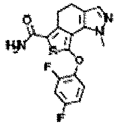 | | |
| 8 | 4-((4-Fluorophenyl)sulfonyl)-3-(2-(imidazo[1,2-b]pyridazin-6-ylsulfanyl)ethyl)-3,4-dihydroquinoxalin-2(1H)-one | 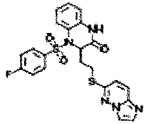 | | |
| 9 | 2-(Benzylamino)-4-(1H-pyrrolo[2,3-b]pyridine-3-yl)benzamide | 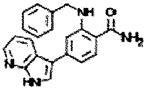 | | **343.2** |
| 10 | 3-(3-(Benzyloxy)phenyl)-1H-pyrrolo[2,3-b]pyridine | 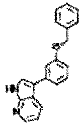 | | |
| 11 | 4-(4-(2,3-Dihydro-1,4-benzodioxin-6-yl)-1H-pyrazol-3-yl)benzene-1,3-diol | 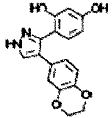 | | |
| 12 | N-Butyl-8-(4-methoxyphenyl)-1,6-naphthyridine-2-carboxamide | 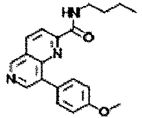 | **CF₃COOH** | |
| 13 | 8-(4-Methylphenyl)-N, N-dipropyl-1,6-naphthyridine-2-carboxamide | 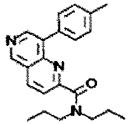 | **CF₃COOH** | |

Examples of the CDK8/19 inhibitor that may be used in the present invention can include, but are not particularly limited to, the following compounds and salts thereof: Senexin A (Cas. No. 1780390-76-2), UNC10112785 (N-(4-Isopropylphenyl)-4-(pyrazolo[1,5-b]pyridazin-3-yl)pyrimidin-2-amine), SEL120-34A (Cas. No. 1609452-30-3), MSC2530818 (Cas. No. 1883423-59-3), Cortistatin A (Cas. No. 882976-95-6), CDK8/19-IN-1 (Cas. No. 1818427-07-4), CDK8-IN-I (4- (4-Methylnaphthalen-1-yl)-2-((3-morpholinophenyl)sulfonamido)benzoic acid), CDK8-IN-II (Cas. No. 1613638-82-6), CDK8-IN-3 (Cas. No. 1884500-15-5), CDK8-IN-III (Cas. No. 1814891-79-6), CDK8-IN-4 (Cas. No. 1613638-82-6), CDK8-IN-IV ((Z)-16-chloro-11,21,25,61-tetramethyl-11H,21H,61H-10-oxa-4,8-dithia-1(7,3)-indola-2(4,3),6(3,5)-dipyrazola-9(3,1)-naphthalenacyclotridecaphane-12-carboxylic acid), CDK8/19-IN-4k (Cas. No. 1818410-84-2), CDK8/19-IN-18 (Cas. No. 1879980-97-8), CDK8/19-IN-32 (7-Amino-4-(4-chlorophenoxy)thieno[2,3-c]pyridine-2-carboxamide), BRD6989 (Cas. No. 642008-81-9), CCT251921 (Cas. No. 1607837-31-9), CCT251545 (Cas. No. 1661839-45-7), Wogonin (Cas. No. 632-85-9), JH-XVI-178 (Cas. No. 2648453-53-4), LY2857785 (Cas. No. 1619903-54-6), AS2863619 (Cas. No. 2241300-51-4), 3MB-PP1 (Cas. No. 956025-83-5), CDK8/19i (Cas. No. 923596-52-5), H-8 (hydrochloride) (Cas. No. 294646-77-8), JH-VIII-49((3S,8S,10R,13S,14S,17S)-17-(isoquinolin-7-yl)-N,N,10,13-tetramethyl-2,3,4,7,8,9,10,11,12,13,14,15,16,17-tetradecahydro-1H-cyclopenta[a]phenanthren-3-amine), JH-XI-10-02 (Cas. No. 2209085-22-1), SNX2-1-108 (Cas. No. 1366002-73-4), W-34 (1-(3,4-Dichloro-phenyl)-3-{1-[5-(4-fluoro-benzyl)-[1,2,4]thiadiazol-3-yl]-piperidin-4-ylmethyl}-urea).

Preferably, the CDK8/19 inhibitor that may be used in the present invention is 2-(4-(4-(isoquinolin-4-yl)phenyl)-1H-pyrazol-1-yl)-N,N-dimethylacetamide (also called "BI-1347" or "1H-pyrazole-1-acetamide, 4-[4-(4-isoquinolinyl)phenyl]-N,N-dimethyl"), 4-((2-(6-(4-methylpiperazine-1-carbonyl)naphthalen-2-yl)ethyl)amino)quinazoline-6-carbonitrile (also called "Senexin B" or "SNX-2-1-165"), or a salt thereof.

In the present invention, these CDK8/19 inhibitors and salts thereof may each be a non-solvate or may be a solvate. The solvate can be formed from a solvent such as ethanol or water. The solvate whose incorporated solvent is water is a hydrate. The hydrate encompasses stoichiometric hydrates as well as hydrates containing various amounts of water.

In the present invention, examples of "salt" include salts with inorganic bases, ammonium salts, salts with organic bases, salts with inorganic acids, salts with organic acids, and salts with basic or acidic amino acids.

Preferred examples of the salts with inorganic bases include alkali metal salts such as sodium salt and potassium salt; alkaline earth metal salts such as calcium salt, magnesium salt, and barium salt; and aluminum salts.

Preferred examples of the salts with organic bases include salts with trimethylamine, triethylamine, pyridine, picoline, ethanolamine, diethanolamine, triethanolamine, dicyclohexylamine, and N,N'-dibenzylethylenediamine.

Preferred examples of the salts with inorganic acids include salts with hydrochloric acid, hydrobromic acid, nitric acid, sulfuric acid, and phosphoric acid.

Preferred examples of the salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid.

Preferred examples of the salts with basic amino acids include salts with arginine, lysine, and ornithine.

Preferred examples of the salts with acidic amino acids include salts with aspartic acid and glutamic acid.

Among these salts, a pharmaceutically acceptable salt is preferred.

The CDK8/19 inhibitor according to the present invention is not limited to the compounds described above, and an antisense oligonucleotide or siRNA against CDK8/19 mRNA, an antibody binding to CDK8/19, a dominant negative CDK8/19 mutant, or the like can also be used as the CDK8/19 inhibitor.

The CDK8/19 inhibitor described above is commercially available or can be synthesized for use according to a known method.

"ALK5 inhibitor" used in the present invention is not particularly limited as long as the ALK5 inhibitor has no mutagenicity and has inhibitory activity against ALK5 (activin receptor-like kinase 5). Any factor that directly or indirectly inhibits the function of ALK5, except for ALK5iII, can be used.

In the present invention, conventionally known "ALK5 inhibitor" can be used. More specifically, examples thereof include, but are not particularly limited to, compounds given below and salts thereof. These compounds may have one or more substituents or their substructures (substituents, rings, etc.) may be partially converted as long as the compounds have ALK5-inhibiting activity.

**[Table 3]**

| Compound name | **IUPAC** Name | Structural formula |
|---|---|---|
| SB431542 | 4-[4-(1,3-Benzodioxol-5-yl)-5-(2-pyridinyl)-1H-imidazol-2-yl]-benzamide | 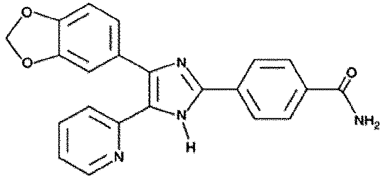 |
| SB525334 | 6-[2-tert-Butyl-5-(6-methylpyridin-2-yl)-1H-imidazol-4-yl]quinoxaline | 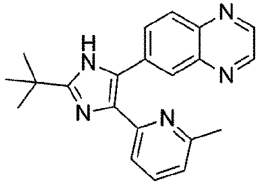 |
| IN1130 | 3-[[5-(6-Methyl-2-pyridinyl)-4-(6-quinoxalinyl)-1H-imidazol-2-yl]methyl]benzamide | 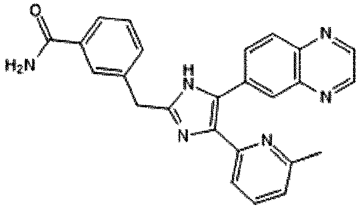 |
| EW-7197 | 2-Fluoro-N-[[5-(6-methylpyridin-2-yl)-4-([1,2,4]triazolo[1,5-a]pyridin-6-yl)-1H-imidazol-2-yl]methyl]aniline | 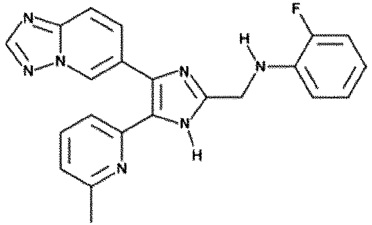 |

Examples of the ALK5 inhibitor that may be used in the present invention can include, but are not particularly limited to, the following compounds and salts thereof: Galunisertib (LY2157299) (Cas. No. 700874-72-2), SB505124 (Cas. No. 694433-59-5), LY364947 (Cas. No. 396129-53-6), K02288 (Cas. No. 1431985-92-0), LDN-214117 (Cas. No. 1627503-67-6), SD-208 (Cas. No. 627536-09-8), ML347 (LDN193719) (Cas. No. 1062368-49-3), LDN-212854 (Cas. No. 1432597-26-6), DMH1 (Cas. No. 1206711-16-1), Pirfenidone (Cas. No. 53179-13-8), LY 3200882 (Cas. No. 1898283-02-7), Alantolactone (Cas. No. 546-43-0), SIS3 (Cas. No. 1009104-85-1), Hesperetin (Cas. No. 520-33-2), Dorsomorphin (BML-275) (Cas. No. 866405-64-3), ALK2-IN-2 (Cas. No. 2254409-25-9), San78-130 (Cas. No. 66018-45-9), TGFβRI-IN-1 (Cas. No. 1950628-94-0), Crizotinib-d5 (Cas. No. 1395950-48-7), ALK-IN-1 (Cas. No. 1197958-12-5), A-77-01 (Cas. No. 607737-87-1), TAE-684 (Cas. No. 761439-42-3), D 4476 (Cas. No. 301836-43-1), Galunisertib (Cas. No. 700874-72-2), SM16 (Cas. No. 614749-78-9), N-Acetylpuromycin (Cas. No. 22852-13-7), TGF-β RI Kinase Inhibitor IX (Cas. No. 301836-41-9), TGF-β RI Kinase Inhibitor VII (Cas. No. 666729-57-3), IM-412 (3-(2-chlorobenzyl)-1,7-dimethyl-1H-imidazo[2,1-f]purine-2,4 (3H,8H)-dione), TSP-1 (LSKL, Inhibitor of Thrombospondin) (Cas. No. 283609-79-0), R268712 (Cas. No. 879487-87-3), LY2109761 (Cas. No. 700874-71-1), GW788388 (Cas. No. 452342-67-5), LDN-193189 (Cas. No. 1062368-24-4), A-83-01 (Cas. No. 909910-43-6), ITD1 (Cas. No. 1099644-42-4), IWR1 (Cas. No. 1127442-82-3), YN968D1 (Cas. No. 811803-05-1), BFH-772 (Cas. No. 890128-81-1), Regorafenib (Cas. No. 755037-03-7).

Preferably, the ALK5 inhibitor that may be used in the present invention is 4-[4-(1,3-benzodioxol-5-yl)-5-(2-pyridinyl)-1H-imidazol-2-yl]-benzamide, 6-[2-tert-butyl-5-(6-methylpyridin-2-yl)-1H-imidazol-4-yl]quinoxaline, 3-[[5-(6-methyl-2-pyridinyl)-4-(6-quinoxalinyl)-1H-imidazol-2-yl]methyl]benzamide, 2-fluoro-N-[[5-(6-methylpyridin-2-yl)-4-([1,2,4]triazolo[1,5-a]pyridin-6-yl)-1H-imidazol-2-yl]methyl]aniline, or a salt thereof.

In the present invention, these ALK5 inhibitors and salts thereof may each be a non-solvate or may be a solvate. Examples of "solvate" and "salt" include those defined above.

The ALK5 inhibitor according to the present invention is not limited to the compounds described above, and an antisense oligonucleotide or siRNA against mRNA of ALK5 or its receptor, an antibody binding to ALK5 or its receptor, a dominant negative ALK5 mutant, a dominant negative ALK5 receptor mutant, or the like can also be used as the ALK5 inhibitor.

The ALK5 inhibitor described above is commercially available or can be synthesized for use according to a known method.

Preferably, in the present invention, the medium contains a combination of one or more ALK5 inhibitors selected from 4-[4-(1,3-benzodioxol-5-yl)-5-(2-pyridinyl)-1H-imidazol-2-yl]-benzamide, 6-[2-tert-butyl-5-(6-methylpyridin-2-yl)-1H-imidazol-4-yl]quinoxaline, 3-[[5-(6-methyl-2-pyridinyl)-4-(6-quinoxalinyl)-1H-imidazol-2-yl]methyl]benzamide, 2-fluoro-N-[[5-(6-methylpyridin-2-yl)-4-([1,2,4]triazolo[1,5-a]pyridin-6-yl)-1H-imidazol-2-yl]methyl]aniline, and their salts, and one or more CDK8/19 inhibitors selected from 2-(4-(4-(isoquinolin-4-yl)phenyl)-1H-pyrazol-1-yl)-N,N-dimethylacetamide, 4-((2-(6-(4-methylpiperazine-1-carbonyl)naphthalen-2-yl)ethyl)amino) quinazoline-6-carbonitrile and their salts. More preferably, in the present invention, the medium contains a combination of 4-[4-(1,3-benzodioxol-5-yl)-5-(2-pyridinyl)-1H-imidazol-2-yl]-benzamide or a salt thereof as the ALK5 inhibitor, and 4-((2-(6-(4-methylpiperazine-1-carbonyl)naphthalen-2-yl)ethyl)amino) quinazoline-6-carbonitrile or 2-(4-(4-(isoquinolin-4-yl)phenyl)-1H-pyrazol-1-yl)-N,N-dimethylacetamide or a salt thereof as the CDK8/19 inhibitor.

"Insulin-positive cell population" according to the present invention means a cell population comprising insulin-positive cells obtained by the induction of differentiation from pluripotent stem cells. "Insulin-positive cells" means cells characterized in that the expression of a marker of insulin is found. "Insulin-positive cells" are cells that may express a marker of NK6 homeobox 1 (NKX6.1) and preferably express both markers of insulin and NKX6.1 (that is, insulin-positive and NKX6.1-positive cells). The insulin-positive cells may include β cells.

"Insulin-positive cell population" according to the present invention can be obtained by culturing and differentiating a pancreatic progenitor cell population obtained by the induction of differentiation from pluripotent stem cells, or a cell population at a later stage of differentiation in a medium containing no ALK5iII and containing a factor having ALK5-inhibiting activity and CDK8/19-inhibiting activity. The insulin-positive cell population may include other cells (for example, endocrine progenitor cells; other pancreatic hormone-producing cells expressing at least one of the markers glucagon, somatostatin, and pancreatic polypeptide (α cells, δ cells, etc.); CHGA-positive cells; Ki67-positive cells; and CHGA-negative cells), in addition to the insulin-positive cells.

In the present invention, "pancreatic progenitor cell population or cell population at a later stage of differentiation" means a pancreatic progenitor cell population, an endocrine progenitor cell population, or an insulin-positive cell population, or two or more cell populations selected from a pancreatic progenitor cell population, an endocrine progenitor cell population, and an insulin-positive cell population. Preferably, in the present invention, "pancreatic progenitor cell population or cell population at a later stage of differentiation" means a pancreatic progenitor cell population or an endocrine progenitor cell population, or both of a pancreatic progenitor cell population and an endocrine progenitor cell population.

As used herein, "pluripotency" means the ability to differentiate into tissues and cells having various different shapes and functions and to differentiate into cells of any lineage of the 3 germ layers. "Pluripotency" is different from "totipotency", which is the ability to differentiate into any tissue of the living body, including the placenta, in that pluripotent cells cannot differentiate into the placenta and therefore, do not have the ability to form an individual.

As used herein, "multipotency" means the ability to differentiate into plural and limited numbers of linages of cells. For example, mesenchymal stem cells, hematopoietic stem cells, neural stem cells are multipotent, but not pluripotent.

As used herein, "pluripotent stem cells" refers to embryonic-stem cells (ES cells) and cells potentially having a pluripotency similar to that of ES cells, that is, the ability to differentiate into various tissues (all of the endodermal, mesodermal, and ectodermal tissues) in the living body. Examples of cells having a pluripotency similar to that of ES cells include "induced pluripotent stem cells" (that may be herein also referred to as "iPS cells"). In the present invention, preferably, pluripotent stem cells are human pluripotent stem cells.

Available "ES cells" include murine ES cells, such as various murine ES cell lines established by inGenious, Institute of Physical and Chemical Research (RIKEN), and the like, and human ES cells, such as various human ES cell lines established by National Institutes of Health (NIH), RIKEN, Kyoto University, Cellartis, and the like. For example, available ES cell lines include CHB-1 to CHB-12, RUES1, RUES2, HUES1 to HUES28 from NIH, and the like; H1 and H9 from WiCell Research Institute; and KhES-1, KhES-2, KhES-3, KhES-4, KhES-5, SSES1, SSES2, SSES3 from RIKEN, and the like.

"Induced pluripotent stem cells" refers to cells that are obtained by reprograming mammalian somatic cells or undifferentiated stem cells by introducing particular factors (nuclear reprogramming factors). At present, there are various "induced pluripotent stem cells" and iPS cells established by Yamanaka, et al. by introducing the 4 factors Oct3/4, Sox2, Klf4, and c-Myc into murine fibroblasts (Takahashi K, Yamanaka S., Cell, (2006) 126: 663-676); iPS cells derived from human cells, established by introducing similar 4 factors into human fibroblasts (Takahashi K, Yamanaka S., et al. Cell, (2007) 131: 861-872.); Nanog-iPS cells established by sorting cells using expression of Nanog as an indicator after introduction of the 4 factors (Okita, K., Ichisaka, T., and Yamanaka, S. (2007). Nature 448, 313-317.); iPS cells produced by a method not using c-Myc (Nakagawa M, Yamanaka S., et al. Nature Biotechnology, (2008) 26, 101-106); and iPS cells established by introducing 6 factors in a virus-free way (Okita K et al. Nat. Methods 2011 May; 8(5): 409-12, Okita K et al. Stem Cells. 31 (3) 458-66) may be also used. Also, induced pluripotent stem cells established by introducing the 4 factors OCT3/4, SOX2, NANOG, and LIN28 by Thomson et al. (Yu J., Thomson JA. et al., Science (2007) 318: 1917-1920.); induced pluripotent stem cells produced by Daley et al. (Park IH, Daley GQ.et al., Nature (2007) 451: 141-146); induced pluripotent stem cells produced by Sakurada et al. (Japanese Unexamined Patent Application Publication No. 2008-307007) and the like may be used.

In addition, any of known induced pluripotent stem cells known in the art described in all published articles (for example, Shi Y., Ding S., et al., Cell Stem Cell, (2008) Vol 3, Issue 5, 568-574; Kim JB., Scholer HR., et al., Nature, (2008) 454, 646-650; Huangfu D., Melton, DA., et al., Nature Biotechnology, (2008) 26, No. 7, 795-797) or patents (for example, Japanese Unexamined Patent Application Publication No. 2008-307007, Japanese Unexamined Patent Application Publication No. 2008-283972, US2008-2336610, US2009-047263, WO2007-069666, WO2008-118220, WO2008-124133, WO2008-151058, WO2009-006930, WO2009-006997, WO2009-007852) may be used.

Available induced pluripotent cell lines include various iPS cell lines established by NIH, Institute of Physical and Chemical Research (RIKEN), Kyoto University and the like. For example, such human iPS cell lines include the RIKEN cell lines HiPS-RIKEN-1A, HiPS-RIKEN-2A, HiPS-RIKEN-12A, and Nips-B2 and the Kyoto University cell lines Ff-WJ-18, Ff-I01s01, Ff-I01s02, Ff-I01s04, Ff-I01s06, Ff-I14s03, Ff-I14s04, QHJI01s01, QHJI01s04, QHJI14s03, QHJI14s04, 253G1, 201B7, 409B2, 454E2, 606A1, 610B1, 648A1, CDI cell lines MyCell iPS Cells (21525.102.10A), MyCell iPS Cells (21526.101.10A), and the like.

As used herein, "pancreatic progenitor cell population" means a cell population comprising pancreatic progenitor cells. As used herein, pancreatic progenitor cells are characterized by the expression of the marker NKX6.1 (that is, the cells are NKX6.1-positive). The pancreatic progenitor cells may further express at least one of the markers PDX-1, PTF-1α, GATA4 and SOX9. Preferably, the pancreatic progenitor cells are characterized by the expression of NKX6.1 and PDX-1 (that is, the cells are NKX6.1-positive and PDX-1-positive).

In one embodiment, "pancreatic progenitor cell population" according to the present invention is a cell population that corresponds to a culture after the completion of step 4) or a culture in step 5) in the process of inducing the differentiation of pluripotent stem cells into pancreatic β cells as described below in detail.

"Pancreatic progenitor cell population" according to the present invention comprises pancreatic progenitor cells at a proportion of 30% or more, preferably 40% or more, more preferably 50% or more, further preferably 60% or more, still further preferably 70% or more. The pancreatic progenitor cell population may include other cells (for example, endocrine progenitor cells, insulin-positive cells, Ki67-positive cells, and CHGA-negative cells), in addition to the pancreatic progenitor cells.

The proportion of specific cells in a cell population described herein can be determined on the basis of a known approach capable of calculating the number of cells, such as flow cytometry.

As used herein, "endocrine progenitor cell population" means a cell population comprising endocrine progenitor cells. As used herein, endocrine progenitor cells mean cells characterized by the expression of at least one of the markers CHGA, NeuroD and NGN3 and no expression of a marker of the pancreas-related hormone system (for example, insulin). The endocrine progenitor cells may express a marker such as PAX-4, NKX2.2, Islet-1, or PDX-1.

In one embodiment, "endocrine progenitor cell population" according to the present invention is a cell population that corresponds to a culture after the completion of step 5) or a culture in step 6) in the process of inducing the differentiation of pluripotent stem cells into pancreatic β cells as described below in detail.

"Endocrine progenitor cell population" according to the present invention comprises endocrine progenitor cells at a proportion of 30% or more, preferably 40% or more, more preferably 50% or more, further preferably 60% or more, still further preferably 70% or more. The endocrine progenitor cell population may include other cells (for example, pancreatic progenitor cells, insulin-positive cells, Ki67-positive cells, and CHGA-negative cells), in addition to the endocrine progenitor cells.

It is known that cells having different features depending on the stages of differentiation appear in the process of differentiation of pluripotent stem cells into insulin-positive cells (WO2009/012428 and WO2016/021734). For example, the stages of differentiation can be broadly classified into pluripotent stem cells, definitive endoderm cells, primitive gut tube cells, posterior foregut cells, pancreatic progenitor cells, endocrine progenitor cells, and insulin-positive cells in order from relatively undifferentiated to differentiated forms.

The pancreatic progenitor cell population or the cell population at a later stage of differentiation can be obtained by use of a known approach of inducing the differentiation of pluripotent stem cells into insulin-positive cells. Specifically, each cell population at a stage of differentiation of interest can be obtained using the following steps of induction of differentiation:
step 1) inducing the differentiation of pluripotent stem cells into definitive endoderm cells;
step 2) inducing the differentiation of the definitive endoderm cells into primitive gut tube cells;
step 3) inducing the differentiation of the primitive gut tube cells into posterior foregut cells;
step 4) inducing the differentiation of the posterior foregut cells into pancreatic progenitor cells;
step 5) inducing the differentiation of the pancreatic progenitor cells into endocrine progenitor cells; and
step 6) inducing the differentiation of the endocrine progenitor cells into insulin-positive cells.

Hereinafter, each step will be described, though the induction of differentiation into each cell is not limited by these approaches.

### Step 1) Differentiation into definitive endoderm cells

The pluripotent stem cells are first allowed to differentiate into definitive endoderm cells. Methods for inducing the definitive endoderm from pluripotent stem cells have already been known, and any of the methods may be used. Preferably, the pluripotent stem cells are cultured in a medium containing activin A, more preferably a medium containing activin A, a ROCK inhibitor, and a GSK3β inhibitor, to thereby differentiate into definitive endoderm cells. The number of cells at the start of culture is not particularly limited and is 22000 to 150000 cells/cm², preferably 22000 to 100000 cells/cm², more preferably 22000 to 80000 cells/cm². The culture period is 1 day to 4 days, preferably 1 day to 3 days, particularly preferably 3 days.

The culture temperature is not particularly limited, and the culture is performed at 30 to 40°C (for example, 37°C). The concentration of carbon dioxide in a culture container is on the order of, for example, 5%. The culture may be performed by any of two-dimensional culture and three-dimensional culture.

The medium used in this step may be a basal medium for use in the culture of mammalian cells, such as RPMI 1640 medium, MEM medium, iMEM medium, DMEM/F12 medium, Improved MEM Zinc Option medium, Improved MEM/1% B-27/Penicillin Streptomycin medium, or MCDB131/20 mM Glucose/NaHCO₃/FAF-BSA/ITS-X/GlutaMAX(TM)/ascorbic acid/Penicillin Streptomycin medium.

The concentration of the activin A in the medium is usually 30 to 200 ng/mL, preferably 50 to 150 ng/mL, more preferably 70 to 120 ng/mL, particularly preferably about 100 ng/mL.

In another embodiment, the activin A can be contained at a low dose, for example, in an amount of 5 to 100 ng/mL, preferably 5 to 50 ng/mL, more preferably 5 to 10 ng/mL, in the medium.

In a further alternative embodiment, the concentration of the activin A in the medium is about 0.1 to 100 ng/mL, preferably about 1 to 50 ng/mL, more preferably about 3 to 10 ng/mL.

The concentration of the GSK3β inhibitor in the medium is appropriately set depending on the type of the GSK3β inhibitor used. For example, in the case of using CHIR99021 as the GSK3β inhibitor, its concentration is usually 2 to 5 µM, preferably 2 to 4 µM, particularly preferably about 3 µM.

The concentration of the ROCK inhibitor in the medium is appropriately set depending on the type of the ROCK inhibitor used. For example, in the case of using Y27632 as the ROCK inhibitor, its concentration is usually 5 to 20 µM, preferably 5 to 15 µM, particularly preferably about 10 µM.

The medium can be further supplemented with insulin. The insulin can be contained in an amount of 0.01 to 20 µM, preferably 0.1 to 10 µM, more preferably 0.5 to 5 µM, in the medium. The concentration of the insulin in the medium may be, but is not limited to, the concentration of insulin contained in added B-27 supplement.

In a particular embodiment, the cells are cultured for 1 day in a medium containing activin A, a ROCK inhibitor, and a GSK3β inhibitor and then further cultured for 2 days in a medium containing only activin A with the medium replaced with a fresh one every day. Alternatively, in the presence of a low dose of activin A, the pluripotent stem cells can be subjected to first culture in a medium containing 0.01 to 20 µM insulin and subsequently subjected to second culture in an insulin-free medium, for production.

### Step 2) Differentiation into primitive gut tube cells

The definitive endoderm cells obtained in step 1) are further cultured in a medium containing a growth factor to induce their differentiation into primitive gut tube cells. The culture period is 2 days to 8 days, preferably about 4 days.

The culture temperature is not particularly limited, and the culture is performed at 30 to 40°C (for example, 37°C). The concentration of carbon dioxide in a culture container is on the order of, for example, 5%. The culture may be performed by any of two-dimensional culture and three-dimensional culture.

A basal medium for use in the culture of mammalian cells can be used as culture medium, as in step 1). The medium may be appropriately supplemented with a serum replacement, a vitamin, an antibiotic, and the like, in addition to the growth factor.

The growth factor is preferably EGF, KGF, and/or FGF10, more preferably EGF and/or KGF, further preferably KGF.

The concentration of the growth factor in the medium is appropriately set depending on the type of the growth factor used and is usually about 0.1 nM to 1000 µM, preferably about 0.1 nM to 100 µM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM). For example, in the case of using KGF as the growth factor, its concentration is usually 5 to 150 ng/mL, preferably 30 to 100 ng/mL, particularly preferably about 50 ng/mL.

### Step 3) Differentiation into posterior foregut cells

The primitive gut tube cells obtained in step 2) are further cultured in a medium containing a growth factor, cyclopamine, noggin, and the like to induce their differentiation into posterior foregut cells. The culture period is 1 day to 5 days, preferably about 2 days. The culture may be performed by any of two-dimensional culture and three-dimensional culture.

The culture temperature is not particularly limited, and the culture is performed at 30 to 40°C (for example, 37°C). The concentration of carbon dioxide in a culture container is on the order of, for example, 5%.

As in step 1), a basal medium for use in the culture of mammalian cells can be used as culture medium. The medium may be appropriately supplemented with a serum replacement, a vitamin, an antibiotic, and the like, in addition to the growth factor.

The growth factor is preferably EGF, KGF, and/or FGF10, more preferably EGF and/or KGF, further preferably KGF.

The concentration of the growth factor in the medium is appropriately set depending on the type of the growth factor used and is usually about 0.1 nM to 1000 µM, preferably about 0.1 nM to 100 µM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM). For example, in the case of using KGF as the growth factor, its concentration is usually 5 to 150 ng/mL, preferably 30 to 100 ng/mL, particularly preferably about 50 ng/mL.

The concentration of the cyclopamine in the medium is not particularly limited and is usually 0.5 to 1.5 µM, preferably 0.3 to 1.0 µM, particularly preferably about 0.5 µM.

The concentration of the noggin in the medium is not particularly limited and is usually 10 to 200 ng/mL, preferably 50 to 150 ng/mL, particularly preferably about 100 ng/mL.

### Step 4) Differentiation into pancreatic progenitor cells

The posterior foregut cells obtained in step 3) are further cultured in a medium containing a factor having CDK8/19-inhibiting activity, preferably a medium containing a factor having CDK8/19-inhibiting activity and a growth factor, to induce their differentiation into pancreatic progenitor cells. The culture period is 2 days to 10 days, preferably about 5 days. The culture may be performed by any of two-dimensional culture and three-dimensional culture.

In the case of two-dimensional culture, according to the previous report (Toyoda et al., Stem cell Research (2015) 14, 185-197), the posterior foregut cells obtained in step 3) are treated with 0.25% trypsin-EDTA solution and dispersed in the solution by pipetting to obtain a cell dispersion, and the obtained dispersion is subjected to centrifugal separation. Recovered cells are resuspended in a small amount of a fresh medium and the cell suspension is reseeded to a fresh medium of step 4).

As in step 1), a basal medium for use in the culture of mammalian cells can be used as culture medium. The medium may be appropriately supplemented with a serum replacement, a vitamin, an antibiotic, and the like, in addition to the growth factor.

Each of the compounds mentioned above or salts thereof can be used as the factor having CDK8/19-inhibiting activity. The amount of the factor added to the medium is appropriately determined according to the compound or the salt thereof used and is usually about 0.00001 µM to 5 µM, preferably 0.00001 µM to 1 µM. The concentration of the factor having CDK8/19-inhibiting activity in the medium is preferably a concentration that attains inhibitory activity of 50% or more for CDK8/19.

The growth factor is preferably EGF, KGF, and/or FGF10, more preferably KGF and/or EGF, further preferably KGF and EGF.

The concentration of the growth factor in the medium is appropriately set depending on the type of the growth factor used and is usually about 0.1 nM to 1000 µM, preferably about 0.1 nM to 100 µM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM). For example, in the case of using KGF and EGF as the growth factor, the concentration of EGF is usually 5 to 150 ng/mL, preferably 30 to 100 ng/mL, particularly preferably about 50 ng/mL, and the concentration of KGF is usually 10 to 200 ng/mL, preferably 50 to 150 ng/mL, particularly preferably about 100 ng/mL.

Culture on the first day in step 4) may be performed in the presence of a ROCK inhibitor, and culture on the following days may be performed in a medium containing no ROCK inhibitor.

The medium may also contain a protein kinase C (PKC) activator. PdBU (PKC activator II), TPB (PKC activator V), or the like is used as the PKC activator, though the PKC activator is not limited thereto. The concentration of the PKC activator to be added is about 0.1 to 100 ng/ml, preferably about 1 to 50 ng/ml, more preferably about 3 to 10 ng/ml.

The medium may also be supplemented with dimethyl sulfoxide and/or activin (1 to 50 ng/ml).

In any of the steps, the medium may be supplemented with a serum replacement (for example, B-27 supplement, ITS-G), in addition to the components described above. Also, an amino acid, L-glutamine, GlutaMAX (product name), a non-essential amino acid, a vitamin, nicotinamide, an antibiotic (for example, Antibiotic-Antimycotic, penicillin, streptomycin, or a mixture thereof), an antimicrobial agent (for example, amphotericin B), an antioxidant, pyruvic acid, a buffer, inorganic salts, and the like may be added thereto, if necessary. In the case of adding an antibiotic to the medium, its concentration in the medium is usually 0.01 to 20% by weight, preferably 0.1 to 10% by weight. The culture may be performed by any of two-dimensional culture and three-dimensional culture.

In the case of two-dimensional culture, the cell culture is performed by adherent culture without the use of feeder cells. For the culture, a culture container, for example, a dish, a flask, a microplate, or a cell culture sheet such as OptiCell (product name) (Nunc), is used. The culture container is preferably surface-treated in order to improve adhesiveness to cells (hydrophilicity), or coated with a substrate for cell adhesion such as collagen, gelatin, poly-L-lysine, poly-D-lysine, laminin, fibronectin, Matrigel (for example, BD Matrigel (Nippon Becton Dickinson Company, Ltd.)), or vitronectin. The culture container is preferably a culture container coated with type I-collagen, Matrigel, fibronectin, vitronectin or poly-D-lysine, more preferably a culture container coated with Matrigel or poly-D-lysine.

The culture temperature is not particularly limited, and the culture is performed at 30 to 40°C (for example, 37°C). The concentration of carbon dioxide in a culture container is on the order of, for example, 5%.

The pancreatic progenitor cells obtained in step 4) can be further purified using a known surface marker glycoprotein 2 (GP2) or the like. The purification can be performed by a method known per se, for example, using anti-GP2 antibody-immobilized beads.

### Step 5) Differentiation into endocrine progenitor cells

The pancreatic progenitor cells obtained in step 4) are further cultured in a medium containing a growth factor to induce their differentiation into endocrine progenitor cells. The culture may be performed by any of two-dimensional culture and three-dimensional culture. In the case of two-dimensional culture, the pancreatic progenitor cells obtained in step 4) are treated with 0.25% trypsin-EDTA solution and dispersed in the solution by pipetting to obtain a cell dispersion, and the obtained dispersion is subjected to centrifugal separation. Recovered cells are resuspended in a small amount of a fresh medium and the cell suspension is reseeded to a fresh medium of step 5). The culture period is 2 days to 3 days, preferably about 2 days.

As in step 1), a basal medium for use in the culture of mammalian cells can be used as culture medium. The medium is supplemented with SANT1, retinoic acid, ALK5 inhibitor II, T3, and LDN according to the previous report (Nature Biotechnology 2014; 32: 1121-1133) and may be appropriately further supplemented with a Wnt inhibitor, a ROCK inhibitor, FGF (preferably FGF2), a serum replacement, a vitamin, an antibiotic, and the like. In the present invention, ALK5 inhibitor II which has heretofore been used in the step 5) is substantially absent or preferably, is not used, because the medium is allowed to contain the aforementioned factor having ALK5-inhibiting activity and CDK8/19-inhibiting activity in the step 5).

The culture is performed by nonadherent culture without the use of feeder cells. For the culture, a dish, a flask, a microplate, a porous plate (Nunc), or the like, or a bioreactor is used. The culture container is preferably surface-treated in order to decrease adhesiveness to cells.

The culture temperature is not particularly limited, and the culture is performed at 30 to 40°C (for example, 37°C). The concentration of carbon dioxide in a culture container is on the order of, for example, 5%.

The endocrine progenitor cells obtained in step 5) can be further purified using a known surface marker glycoprotein 2 (GP2) or the like. The purification can be performed by a method known per se, for example, using anti-GP2 antibody-immobilized beads.

### Step 6) Differentiation into insulin-positive cells

The endocrine progenitor cells obtained in step 5) are further cultured in a medium containing a growth factor to induce their differentiation into insulin-positive cells. The culture period is 10 days to 30 days, preferably about 10 to 20 days.

As in step 1), a basal medium for use in the culture of mammalian cells can be used as culture medium. The medium is supplemented with ALK5 inhibitor II, T3, LDN, γ-secretase inhibitor XX, γ-secretase inhibitor RO, N-cysteine, an AXL inhibitor, and ascorbic acid according to the previous report (Nature Biotechnology 2014; 32: 1121-1133) and may be appropriately further supplemented with a Wnt inhibitor, a ROCK inhibitor, FGF (preferably FGF2), a serum replacement, a vitamin, an antibiotic, and the like. For example, the medium may be supplemented with ALK5 inhibitor II, T3, LDN, γ-secretase inhibitor RO, and ascorbic acid or may be supplemented with T3, ALK5 inhibitor II, ZnSO₄, heparin, N-acetylcysteine, Trolox, and R428. In the present invention, ALK5 inhibitor II which has heretofore been used in the step 6) is substantially absent or preferably, is not used, because the medium is allowed to contain the aforementioned factor having ALK5-inhibiting activity and CDK8/19-inhibiting activity in the step 6).

The culture may be performed by any of two-dimensional culture and three-dimensional culture. The culture does not employ feeder cells. Three-dimensional culture is performed by nonadherent culture. For the culture, a dish, a flask, a microplate, a porous plate (Nunc), or the like, or a bioreactor is used. The culture container is preferably surface-treated in order to decrease adhesiveness to cells.

The culture temperature is not particularly limited, and the culture is performed at 30 to 40°C (for example, 37°C). The concentration of carbon dioxide in a culture container is on the order of, for example, 5%.

In the present invention, "medium containing a factor having ALK5-inhibiting activity and CDK8/19-inhibiting activity" can contain the factor having ALK5-inhibiting activity and CDK8/19-inhibiting activity in any amount capable of inhibiting ALK5 activity and CDK8/19 activity. When the factor is a combination of a plurality of factors consisting of an ALK5 inhibitor and a CDK8/19 inhibitor, the ALK5 inhibitor and the CDK8/19 inhibitor can be contained in any amount capable of inhibiting the activity of each target in the medium.

The ALK5 inhibitor can be contained in an amount of, for example, 10 µM or less, 5 µM or less, 4 µM or less, 3 µM or less, 2 µM or less, or 1 µM or less in the medium. The lower limit of the amount of the ALK5 inhibitor added is not particularly limited and can be 0.1 nM or more, 0.2 nM or more, 0.3 nM or more, 0.4 nM or more, or 0.5 nM or more. The amount of the ALK5 inhibitor added is, for example, 10 µM or less and 0.1 nM or more, preferably 5 µM or less and 0.1 nM or more, more preferably 1 µM or less and 0.1 nM or more, for example, less than 4 µM and 0.1 nM or more or 3 µM or less and 0.3 nM or more.

The CDK8/19 inhibitor can be contained in an amount of, for example, 10 µM or less, 5 µM or less, 4 µM or less, 3 µM or less, 2 µM or less, or 1 µM or less in the medium. The lower limit of the amount of the CDK8/19 inhibitor added is not particularly limited and can be 0.1 nM or more, 0.2 nM or more, 0.3 nM or more, 0.4 nM or more, or 0.5 nM or more. The amount of the CDK8/19 inhibitor added is, for example, 10 µM or less and 0.1 nM or more, preferably 5 µM or less and 0.1 nM or more, more preferably 1 µM or less and 0.1 nM or more, for example, less than 1 µM and 0.1 nM or more.

The culture of the pancreatic progenitor cell population obtained by the induction of differentiation from pluripotent stem cells, or the cell population at a later stage of differentiation in the medium containing the factor having ALK5-inhibiting activity and CDK8/19-inhibiting activity can be performed for at least 12 hours, preferably 24 hours or longer, 2 days or longer, 4 days or longer, 8 days or longer, 10 days or longer, or 15 days or longer. The culture in the medium containing the factor is preferably performed for 4 days or longer. The medium containing the factor may be replaced and can be replaced with a medium supplemented with the factor, having the same or different composition as or from that before the replacement, according to the culture schedule.

The pancreatic progenitor cell population obtained by the induction of differentiation from pluripotent stem cells, or the cell population at a later stage of differentiation can be subjected to the step of culture and differentiation of the cell population in a medium containing a factor having ALK5-inhibiting activity and CDK8/19-inhibiting activity. As used herein, "culture and differentiation in a medium containing a factor having ALK5-inhibiting activity and CDK8/19-inhibiting activity" includes the case of performing the step of culture in the medium containing the factor and the step of differentiation into the cell population of interest at the same time, the case of performing culture in the medium containing the factor, followed by the step of differentiation into the cell population of interest, and the case of subjecting the cell population to the step of differentiation into the cell population of interest, followed by the step of culture in the medium containing the factor. Thus, the medium containing the factor having ALK5-inhibiting activity and CDK8/19-inhibiting activity and the medium for use in the differentiation of the cell population may be separate media, or the medium for use in the step of differentiation may be further supplemented with the factor having ALK5-inhibiting activity and CDK8/19-inhibiting activity.

In one embodiment of the present invention, the factor having ALK5-inhibiting activity and CDK8/19-inhibiting activity is contained in a medium in step 5 or later, that is, a medium in step 5 or a medium in step **6,** or a medium in step 5 and a medium in step **6,** and allowed to act on the cells, in the process of inducing the differentiation of pluripotent stem cells into insulin-positive cells.

The insulin-positive cell population obtained by the present invention can be induced a differentiation or matured into a cell population comprising pancreatic β cells (hereinafter, referred to as "pancreatic β cell population"). As used herein, "pancreatic β cells" means cells more mature than "insulin-positive cells" and specifically means cells characterized by expressing at least one of the markers MAFA, UCN3, and IAPP, which are maturation markers of pancreatic β cells, or by a reaction to increase insulin secretion by glucose stimulation. The pancreatic β cell population may include other cells (for example, insulin-positive cells, Ki67-positive cells and CHGA-negative cells), in addition to the pancreatic β cells.

The pancreatic β cell population can be obtained by the differentiation and/or maturation of the insulin-positive cell population. Preferably, the pancreatic β cell population can be obtained by the *in vivo* differentiation and/or maturation of the insulin-positive cell population in an animal.

"Animal" is preferably a mammal. Examples thereof include humans, nonhuman primates, pigs, cattle, horses, sheep, goats, llamas, dogs, cats, rabbits, mice, and guinea pigs. A human is preferred.

The transplantation is preferably performed to an *in vivo* region where the cell population can be fixed at a given position, and can be performed, for example, subcutaneously, intraperitoneally, to the peritoneal epithelium, to the greater omentum, to a fat tissue, to a muscle tissue, or beneath the capsule of each organ such as the pancreas or the kidney, in the animal. The number of cells to be transplanted may vary depending on factors such as the stage of differentiation of the cells to be transplanted, the age and body weight of a recipient, the size of a transplantation site, and the severity of a disease and is not particularly limited. For example, the number of cells can be on the order of 10 × 10⁴ cells to 10 × 10¹¹ cells. The transplanted cell population is induced to differentiate in an *in vivo* environment and can thereby differentiate into the cell population of interest, preferably a pancreatic β cell population, which may then be recovered or may be indwelled *in vivo* as it is.

For the transplantation, the cell population may be embedded in a gel containing a biocompatible material and then transplanted. For example, the cell population embedded in the gel containing a biocompatible material may be enclosed in a device such as a capsule, a bag, or a chamber and transplanted into a living body.

As used herein, "embedding" means that an endocrine progenitor cell population or a cell population at a later stage of differentiation is contained in a scattered manner in the gel containing a biocompatible material.

As used herein, "biocompatible material" means an arbitrary material that induces neither marked immune response nor harmful biological reaction (for example, toxic reaction and blood coagulation) when transplanted into a living body and indwelled for a short period or a long period. Also, "biocompatible material" is preferably a biodegradable material. Examples of such a material include polylactic acid (PLA), polycaprolactone (PCL), polyurethane (PU), polyethylene glycol (PEG), polyhydroxyethyl methacrylate, polyglycolic acid (PGA), poly(lactic-co-glycolic acid) (PLGA), poly(3-hydroxybutyrate-co-hydroxyvalerate) (PHBV), poly(ethylene-co-vinyl acetate) (PEVA), polyacrylamide, polyethylene oxide, polyethyleneamine, polyhydroxybutyric acid, poly(N-vinylpyrrolidone), polyvinyl alcohol, polypropylene fumarate, polyacrylic acid, poly-e-caprolactone, polymethacrylic acid, polyvinylidene difluoride (PVDF), pectic acid, hyaluronic acid, heparin sulfate, chondroitin sulfate, heparan sulfate proteoglycan, heparin, chitin, chitosan, xanthan, carboxymethylcellulose, carboxymethyl chitosan, alginate, alginic acid ester, collagen, cellulose, silk fibroin, keratin, gelatin, fibrin, pullulan, laminin, gellan, silicon, urethane, elastin and modified forms thereof, and combinations thereof. The surface of "biocompatible material" may be modified (for example, coated with a substrate for cell adhesion (collagen, gelatin, poly-L-lysine, poly-D-lysine, laminin, fibronectin, Matrigel, vitronectin, etc.)) so as to permit cell adhesion or may be engineered with a functional group (for example, an amino group, a carboxyl group, a hydroxy group, a methacrylic acid group, and an acrylic acid group) known to control cell proliferation, differentiation, or functions, if necessary. In a particular embodiment, alginate or alginic acid ester can be suitably used as "biocompatible material".

The alginate can be a water-soluble salt, and a metal salt, an ammonium salt, or the like can be used. For example, sodium alginate, calcium alginate, or ammonium alginate can be suitably used.

The alginic acid ester (also referred to as propylene glycol alginate) is a derivative in which propylene glycol is bonded to the carboxyl group of alginic acid through an ester bond.

The ratio of mannuronic acid to guluronic acid (M/G ratio) contained in the alginate is arbitrary. In general, in the case of M > G, a highly flexible gel can be formed. In the case of M < G, a strong gel can be formed. In the present invention, alginate containing guluronic acid at a proportion of 10 to 90%, 20 to 80%, 30 to 70%, or 40 to 60% can be used.

The gel can be prepared using alginate or alginic acid ester in accordance with a known approach (WO2010/032242 and WO2011/154941) and can be obtained by adding a cross-linking agent to a solution of alginate or alginic acid ester for gelation.

The alginate or the alginic acid ester can be contained in an amount of 0.05 to 10% by weight, preferably 0.1 to 5% by weight, more preferably 0.5 to 3% by weight, in a solvent. The solvent can be any solvent capable of dissolving the alginate or the alginic acid ester, and water, physiological saline, or the like can be used.

The cross-linking agent can be any cross-linking agent that can allow a solution of alginate or alginic acid ester to gelate, and is not particularly limited. A polyvalent metal cation can be used. The polyvalent metal cation is preferably a divalent metal cation, more preferably a calcium ion, a strontium ion, or a barium ion. The cross-linking agent can be used in the form of a salt. In the present invention, at least one member selected from calcium chloride, strontium chloride, and barium chloride can be used as the cross-linking agent.

The gel containing alginate or alginic acid ester can contain a nanofiber. The nanofiber is a natural or synthetic fiber having a diameter of a nanometer order. Examples of the natural nanofiber include nanofibers containing one or more of collagen, cellulose, silk fibroin, keratin, gelatin, and polysaccharides such as chitosan. Examples of the synthetic nanofiber include polylactic acid (PLA), polycaprolactone (PCL), polyurethane (PU), poly(lactide-co-glycolide) (PLGA), poly(3-hydroxybutyrate-co-hydroxyvalerate) (PHBV), and poly(ethylene-co-vinylacetate) (PEVA). The nanofiber can be contained in an amount of less than 1% by weight, for example, 0.9% by weight, 0.8% by weight, 0.7% by weight, 0.6% by weight, 0.5% by weight, or less than the amount, in the gel containing alginic acid. The lower limit of the amount of the nanofiber contained in the gel containing alginate or alginic acid ester is not particularly limited and can be 0.05% by weight or more, preferably 0.1% by weight or more.

The embedding of the cell population in the gel containing alginate or alginic acid ester can be performed by an arbitrary approach and can be performed, for example, by mixing the cell population into a solution of alginate or alginic acid ester and gelating it, though the embedding is not particularly limited thereto.

The cell population can be contained in an amount selected from 1 × 10⁴ cells to 1 × 10⁹ cells/mL, preferably 1 × 10⁷ cells to 1 × 10⁸ cells/mL, in the solution of alginate or alginic acid ester.

The gelation of the solution of alginate or alginic acid ester containing the cell population can be performed by adding a cross-linking agent to the solution. The amount of the cross-linking agent added can be an amount selected from 0.1 to 5% by weight, for example, 0.1 to 1% by weight, with respect to the solution. The gelation can be performed in a container having a predetermined configuration and/or shape for use in cell culture or cell transplantation, or in a mold designed so as to obtain a gel adapted to the container.

Alternatively, the gelation may be performed by forming a gel capsule containing alginic acid in accordance with a known approach (WO2010/010902). Specifically, the solution of alginate or alginic acid ester containing the cell population may be added dropwise to a solution of a cross-linking agent for gelation. The size of liquid droplets can be adjusted according to the shape of a nozzle for dropwise addition or a dropwise addition method, and by extension, the size of the gel capsule containing alginic acid can be defined. The dropwise addition method is not particularly limited and can be performed by an approach such as an air spray method, an airless spray method, or a static spray method. The size of the gel capsule containing alginic acid is not particularly limited and can be a diameter of 5 mm or smaller, 1 mm or smaller, or 500 µm or smaller. The cross-linking agent solution can contain the cross-linking agent in an amount selected from 0.1 to 10% by weight, for example, 0.1 to 5% by weight.

The insulin-positive cell population obtained by the present invention may be indwelled as it is and used as insulin-producing and/or -secreting cells, when transplanted into a living body of an animal and differentiated in the living body of the animal.

The insulin-positive cell population obtained by the present invention is transplanted as it is or in a capsule form to an affected area and is thereby useful as a cell medicine for treating diabetes mellitus, particularly, type I diabetes mellitus.

The insulin-positive cell population obtained by the present invention may be a prodrug. As used herein, the prodrug refers to a cell population that is differentiated after transplantation into a living body and converted to cells having a function of treating a disease.

The insulin-positive cell population obtained by the present invention has low toxicity (for example, acute toxicity, chronic toxicity, genetic toxicity, reproductive toxicity, cardiotoxicity, and carcinogenicity) and can be safely administered as it is or in the form of a pharmaceutical composition containing the cell population mixed with a pharmacologically acceptable carrier, etc. to a mammal (for example, a mouse, a rat, a hamster, a rabbit, a cat, a dog, cattle, sheep, a monkey, and a human).

### 3. Differentiation medium

The present invention provides a differentiation medium for a pancreatic progenitor cell population or a cell population at a later stage of differentiation, comprising a factor having ALK5-inhibiting activity and CDK8/19-inhibiting activity and comprising substantially no ALK5iII.

The differentiation medium of the present invention can be used to induce differentiation of a pancreatic progenitor cell population or a cell population at a later stage of differentiation. The differentiation medium of the present invention can be used in step 5) or step 6) in the aforementioned method for inducing the differentiation of pluripotent stem cells into insulin-positive cells.

The differentiation medium of the present invention comprises the factor having ALK5-inhibiting activity and CDK8/19-inhibiting activity in any amount capable of inhibiting ALK5 activity and CDK8/19 activity in a basal medium for use in the culture of mammalian cells, such as RPMI 1640 medium, MEM medium, iMEM medium, DMEM/F12 medium, Improved MEM Zinc Option medium, Improved MEM/1% B-27/Penicillin Streptomycin medium, or MCDB131/20 mM Glucose/NaHCO₃/FAF-BSA/ITS-X/GlutaMAX(TM)/Ascorbic acid/Penicillin Streptomycin medium. When the factor is a combination of a plurality of factors consisting of an ALK5 inhibitor and a CDK8/19 inhibitor, the respective amounts of the ALK5 inhibitor and the CDK8/19 inhibitor contained in the medium are as defined above.

The differentiation medium of the present invention further contains other factors required for each of step 5) and step 6), such as a growth factor, various inhibitors, a serum replacement, an antibiotic, and a vitamin, in addition to the factor having ALK5-inhibiting activity and CDK8/19-inhibiting activity. According to the previous report (Nature Biotechnology 2014; 32: 1121-1133), for example, the medium for use in step 5) can be supplemented with predetermined amounts of SANT1, retinoic acid, T3, LDN, a Wnt inhibitor, a ROCK inhibitor, FGF (preferably FGF2), a serum replacement, a vitamin, an antibiotic, and the like. The medium for use in step 6) can be supplemented with predetermined amounts of T3, LDN, γ-secretase inhibitor XX, γ-secretase inhibitor RO, N-cysteine, an AXL inhibitor, ascorbic acid, a Wnt inhibitor, a ROCK inhibitor, FGF (preferably FGF2), a serum replacement, a vitamin, an antibiotic, ZnSO₄, heparin, N-acetylcysteine, Trolox, R428, and the like.

The differentiation medium of the present invention contains (or comprises) substantially no ALK5iII. "Contain (or comprise) substantially no ALK5iII" described about the medium of the present invention means that the medium does not contain ALK5iII in a form that exerts arbitrary activity including ALK5-inhibiting activity, and does not necessarily mean that the medium does not contain any ALK5iII. Preferably, this phrase means that the medium of the present invention does not contain any ALK5iII.

The differentiation medium of the present invention may be provided in one form of a mixture of a basal medium, the factor having ALK5-inhibiting activity and CDK8/19-inhibiting activity and other factors described above, or may be provided in any combination or in a plurality of separate forms of these components and prepared just before use.

Hereinafter, the present invention will be described with reference to Examples. However, the present invention is not limited by these Examples.

### Examples

### Example 1: Evaluation of mutagenicity of ALK5iII

Fourteen compounds including ALK5iII heretofore added to a differentiation medium for use in each process of inducing the differentiation of pluripotent stem cells into an insulin-positive cell population were analyzed for their mutagenicity using mutagenicity prediction programs for bacteria (Derek and CASE Ultra) based on (quantitative) structure activity relationship ((Q)SAR).

As a result, five compounds including ALK5iII were confirmed to have a structural alert of mutagenicity. These five compounds confirmed to have a structural alert were further subjected to the Ames test and evaluated for the presence or absence of their mutagenicity.

The Ames test was conducted according to a conventionally known approach. That is, each bacterial strain of *Salmonella typhimurium* TA100, TA1535, TA98, and TA1537 strains and an *Escherichia coli* WP2uvrA strain was mixed with each compound with seven concentrations (78.1 to 5,000 µg/plate), preincubated *in vitro* for 20 minutes, and then inoculated, together with added semifluid agar, to a minimal glucose agar medium, followed by solidification and incubation at 37°C for 48 hours. This experiment involved no rat liver S9 fraction because the experiment was aimed at evaluating the mutagenicity of each compound in a cell culture system, not evaluating metabolites obtained after metabolisms by hepatic enzymes. The positive controls used were strong mutagens 9-aminoacridine hydrochloride monohydrate (9-AA, Sigma-Aldrich Co., LLC) and ICR 191, and the negative control used was dimethyl sulfoxide. Positivity was determined when an average number of revertant colonies in any of the bacterial strains was at least twice or more the average number of the negative control.

As a result of the Ames test, only ALK5iII among the five compounds was determined to be positive in the TA1537 strain (with no S9 fraction) and confirmed to have mutagenicity (Table 4 below).

**[Table 4]**

| TA1537, -S9 | | |
|---|---|---|
| **ALK5 i II** | | 2-(3-(Pyridin-3-yl)-1H-pyrazol-4-yl)quinoxaline |
| *µ* g/ plate | # of colony | # of colony |
| 0 (DMSO) | 8 | 9 |
| 78.1 | 8 | 5 |
| 156 | 7 | 9 |
| 313 | 8 | **21** |
| 625 | **26** | **45** |
| 1250 | **74** | **82** |
| 2500 | **672** | **128** |
| 5000 | **2049** | **194** |

| Positive control | | |
|---|---|---|
| 9-AA, 80 *µ* g | 169 | |
| ICR 191, 1.0 *µ* g | | 1804 |

ALK5iII is a commercially available ALK5 inhibitor and is a derivative of naphthyridine (Figure 1). The TA1537 strain is known to have sensitivity to intercalators for which the concerns of mutagenicity are suggested. Accordingly, in order to reveal whether the mutagenicity of ALK5iII would be derived from its planar structure or related to the ability to inhibit ALK5, the same test and evaluation as above were conducted using a structural analog of ALK5iII (2-(3-(pyridin-3-yl)-1H-pyrazol-4-yl)quinoxaline) and two other representative ALK5 inhibitors (SB525334 and SB431542).

As a result, 2-(3-(pyridin-3-yl)-1H-pyrazol-4-yl)quinoxaline exhibited no ALK5-inhibiting activity but was determined to be positive in the Ames test, as in ALK5iII (Figure 1 and Table 4). On the other hand, the other representative ALK5 inhibitors SB525334 and SB431542 exhibited no mutagenicity (Figure 1). These results indicated that the mutagenicity of ALK5iII is likely to be caused by the planar structure of naphthyridine, not the ability to inhibit ALK5.

### Example 2: Evaluation of off-target effect of ALK5iII

In order to identify a nonmutagenic ALK5 inhibitor capable of being used instead of ALK5iII confirmed to have mutagenicity, a total of 30 ALK5 inhibitors were selected from commercially available compounds and a library constructed by the inventors, and these ALK5 inhibitors were analyzed for their mutagenicity using mutagenicity prediction programs for bacteria (Derek and CASE Ultra) in the same manner as above.

As a result, 20 out of the 30 compounds were found to have no known structural feature related to mutagenicity. Next, these nonmutagenic ALK5 inhibitors were tested for whether or not to be able to produce an insulin-positive cell population rich in insulin-producing cells.

In this testing method, the induction of differentiation of human iPS cells (Ff-I14-s04 line) into a pancreatic progenitor cell population was carried out according to the above steps 1)-4), the previous report (Stem Cell Research (2015) 14, 185-197), etc. The induction of differentiation into insulin-positive cells was carried out according to the above step 5), 6), etc.

The pancreatic progenitor cell population obtained by the induction of differentiation from iPS cells was cultured for 2 days in a medium for induction of differentiation (Improved MEM/1% B-27/Penicillin Streptomycin medium) containing differentiation factors (SANT1, retinoic acid, T3, LDN, a Wnt inhibitor, a ROCK inhibitor, and FGF2) as well as ALK5iII (10 µM) or each nonmutagenic ALK5 inhibitor (representative SB525334 (10 µM), SB431542 (10 µM), IN1130 (1 µM), or EW-7197 (1 µM)) and thereby induced to differentiate into an endocrine progenitor cell population.

Subsequently, the endocrine progenitor cell population was cultured for 7 days in a medium for induction of differentiation (Improved MEM/1% B-27/Penicillin Streptomycin medium) containing differentiation factors (T3, LDN, γ-secretase inhibitor RO, and FGF receptor 1 inhibitor PD-166866) as well as ALK5iII (10 µM) or each nonmutagenic ALK5 inhibitor (SB525334 (10 µM), SB431542 (10 µM), IN1130 (1 µM), or EW-7197 (1 µM)) and then, according to the previous report (Nature Biotechnology 2014; 32: 1121-1133), cultured for 4 days in a medium for induction of differentiation (MCDB131/20 mM Glucose/NaHCO₃/FAF-BSA/ITS-X/Glutamax/Penicillin Streptomycin medium) containing T3, LDN, γ-secretase inhibitor RO, N-acetylcysteine, AXL inhibitor R428, ascorbic acid, a ROCK inhibitor, ZnSO₄, heparin, and Trolox as well as ALK5iII (10 µM) or each nonmutagenic ALK5 inhibitor (SB525334 (10 µM), SB431542 (10 µM), IN1130 (1 µM), or EW-7197 (1 µM)) and thereby induced to differentiate into an insulin-positive cell population.

The number of insulin-positive and NKX6.1-positive cells and the number of CHGA-positive and PDX1-positive cells in the insulin-positive cell population obtained by the above method was counted by flow cytometry to determine the respective proportions of the cells in each cell population. The control medium used contained neither ALK5iII nor the nonmutagenic ALK5 inhibitor.

Figure 2 shows the proportion of insulin-positive and NKX6.1-positive cells and the proportion of CHGA-positive and PDX1-positive cells in the insulin-positive cell population obtained by treatment with the medium for induction of differentiation containing ALK5iII or each nonmutagenic ALK5 inhibitor (SB525334, SB431542, IN1130, or EW-7197). Unlike ALK5iII, in the case of using the nonmutagenic ALK5 inhibitor (SB525334, SB431542, IN1130, or EW-7197), both the proportion of insulin-positive and NKX6.1-positive cells and the proportion of CHGA-positive and PDX1-positive cells were equivalent to the control (-), confirming that unlike ALK5iII, the addition of the nonmutagenic ALK5 inhibitor does not promote differentiation into the insulin-positive cells of interest.

Kinase inhibitors generally interact with diverse targets. Therefore, in order to confirm an off-target effect of ALK5iII, its kinase inhibition profile was analyzed. This test was conducted by using a staurosporine derivative bound with BODIPY-FL or Cy5-FL as a fluorescent probe, applying 0.1 µM and 1 µM ALK5iII thereto, and carrying out competitive binding assay on approximately 350 types of recombinant kinases. In this test, pIC₅₀ values are estimated from inhibitory activity values at the two concentrations of 0.1 µM and 1 µM and evaluated as follows: more than 8.0 = beyond the range (strong inhibitory activity is found), 6.0 to 8.0 = inhibitory activity is found, and 6.0 or less = any inhibitory activity is not detected up to the concentration of 1 µM. The results are shown in Table 5 below. ALK5iII was confirmed to have inhibitory activity with an estimate pIC₅₀ value of more than 6 against 11 kinases including CDK8 and CDK19. In the table, 8.00 ± 0.00 for ALK5 and ALK4 represents that an actual pIC₅₀ value was estimated to be more than 8 from the extrapolation of a measurement value.

**[Table 5]**

| Kinase | Estimate pIC₅₀ value |
|---|---|
| | ALK5iII |
| ALK5 | 8.00 ± 0.00 |
| ALK4 | 8.00 ± 0.00 |
| KDR | 7.22 ± 0.10 |
| CDK19 | 7.18 ± 0.50 |
| TGFBR2 | 6.97 ± 0.12 |
| RIPK2 | 6.82 ± 0.05 |
| CDK8 | 6.80 ± 0.40 |
| PKD3 | 6.51 ± 0.06 |
| PKD2 | 6.40 ± 0.08 |
| PKD1 | 6.38 ± 0.19 |
| TNIK | 6.18 ± 0.14 |

Next, the nonmutagenic ALK5 inhibitors (SB431542, SB525334, IN1130, and EW-7197) were also evaluated for their inhibitory activity against these 11 kinases. As a result, inhibitory activity against CDK8 and CDK19 (CDK8/19) was found to be unique to ALK5iII (Figure 3). These results indicate that the inhibition of CDK8/19 may contribute to the promotion of differentiation into insulin-positive cells.

### Example 3: Combination of ALK5 inhibition and CDK8/19 inhibition that mimic effect of ALK5iII

An insulin-positive cell population was produced in the same manner as in the method for producing the insulin-positive cell population described above in "Example 2" except that any of the following (1) to (4) was used as ALK5iII or the nonmutagenic ALK5 inhibitor to be added to the medium:
(1) 10 µM ALK5iII,
(2) 3 µM nonmutagenic ALK5 inhibitor SB431542,
(3) 0.3 µM CDK8/19 inhibitor Senexin B, and
(4) a combination of 3 µM SB431542 and 0.3 µM Senexin B.

These inhibitors were used at concentrations that could cause complete inhibition in time-resolved fluorescence resonance energy transfer (TR-FRET) cell-free dose response assay.

The number of insulin-positive and NKX6.1-positive cells in the insulin-positive cell population obtained by the above method was counted by flow cytometry to determine the respective proportions of the cells in each cell population.

As a result, the proportion of insulin-positive and NKX6.1-positive cells obtained using (4) a combination of SB431542 and Senexin B (SB/Sen) exhibited a higher percentage than that obtained using (1) ALK5iII (Figure 4A), and the total cell yield was equivalent therebetween (Figure 4B).

On the other hand, the proportion of insulin-positive and NKX6.1-positive cells and the total cell yield obtained using (2) the ALK5 inhibitor SB431542 alone exhibited lower values than those obtained using (1) ALK5iII (Figures 4A and 4B).

Also, the proportion of insulin-positive and NKX6.1-positive cells obtained using (3) the CDK8/19 inhibitor Senexin B alone exhibited a higher percentage than that obtained using (1) ALK5iII (Figure 4A), whereas the total cell yield obtained using (3) Senexin B exhibited a lower value than that obtained using (1) ALK5iII (Figure 4B).

Subsequently, in order to compare cell composition among the respective insulin-positive cell populations produced using the inhibitors (1) to (4), single-cell RNA-seq (scRNA-seq) analysis was conducted. A library for scRNA-seq analysis was prepared using Chromium Controller and a preparation kit (Chromium Next GEM Single Cell 3' Kits v3.1) from 10x Genomics, Inc. and sequenced using Hi-seq platform from Illumina, Inc. Data on the obtained UMI counts was analyzed using Cell Ranger and R Package Seurat from 10x Genomics, Inc. and subjected to dimensionality reduction and visualization using PCA and UMAP. Cell clustering based on the results of dimensionality reduction was carried out by clustering based on the shared nearest neighbor method of Seurat.

As a result, cell clusters #1 to #17 were identified in the respective insulin-positive cell populations produced using the inhibitors (1) to (4). Clusters #1, #2, and #9 were found to be similar to β cells, α cells, and pancreatic acinar cells/pancreatic ductal cells, respectively, from the expression patterns of cell identification markers such as insulin and glucagon and analysis based on a generally published database PanglaoDB (https://panglaodb.se/) (O. Franzen, L.M. Gan, J.L.M. Bjorkegren, PanglaoDB: a web server for exploration of mouse and human single-cell RNA sequencing data. Database (Oxford) 2019 (2019)). The cells of clusters #3 and #6 had features specific for enterochromaffin cells. The cells of cluster #12 had high expression levels of insulin and NKX6.1 and on the other hand, did not express maturation markers for β cells such as MAFA and G6PC2. These cells can thereby be discriminated from the cells of cluster #1 adjacent thereto and are therefore considered to be relatively immature β cells. Some of the β cells might be integrated, together with α cells, δ cells, and γ cells, into cluster #0. The cells of cluster #11 exhibited intermediate properties between hepatocytes and pancreatic α cells, and clusters #7 and #15 were most similar to neuron-like cells.

When each cell cluster was compared among the respective insulin-positive cell populations produced using the inhibitors (1) to (4), typical endocrine cells such as estimate β cells, estimate α cells, and estimate enterochromaffin cells (clusters #0, #1, #2, #3, and #6) were found with almost no exception in the insulin-positive cell population produced using each inhibitor of (1) ALK5iII, (3) Senexin B, and (4) a combination of SB431542 and Senexin B and on the other hand, were not found in the insulin-positive cell population produced using (2) the ALK5 inhibitor SB431542. By contrast, some other clusters (#5, #9, #10, #12, and #13) were specialized for the insulin-positive cell population produced using (2) the ALK5 inhibitor SB431542.

Figure 5 shows the percentage of each cluster (#1, #3, #6, and #12) in insulin-positive and NKX6.1-positive cell subpopulations in the respective insulin-positive cell populations produced using the inhibitors (1) to (4). The percentage of the cluster was almost equivalent between the respective insulin-positive cell populations produced using the inhibitors of (1) ALK5iII and (4) a combination of SB431542 and Senexin B (SB/Sen), and was different from that in the insulin-positive cell population produced using each inhibitor of (2) the ALK5 inhibitor SB431542 and (3) the CDK8/19 inhibitor Senexin B.

When gene expression in cluster #1 and #2 (estimate β cells and estimate α cells) was compared between the insulin-positive cell population produced using (1) ALK5iII and the insulin-positive cell population produced using each of the other inhibitors, the number of differentially expressed genes (DEG) between the two samples was obtained as the lowest value in both the clusters between the respective insulin-positive cell populations produced using the inhibitors of (1) ALK5iII, and (4) a combination of SB431542 and Senexin B (Figure 6). These results suggest that the insulin-positive cell population produced using (1) ALK5iII and the insulin-positive cell population produced using (4) a combination of the ALK5 inhibitor and the CDK8/19 inhibitor have similar *in vitro* profiles.

### Example 4: Evaluation of in vivo effectiveness of insulin-positive cell population obtained without use of ALK5iII

An effect on diabetes mellitus was evaluated *in vivo* using an insulin-positive cell population produced using a combination of the ALK5 inhibitor and the CDK8/19 inhibitor (SB/Sen) without the use of ALK5iII.

A low dose of streptozotocin (STZ, 50 mg/kg/day, 5 days, Sigma-Aldrich Co., LLC) was intraperitoneally administered a plurality of times to male NOD.CB17-Prkdcscid/J (NOD-scid) mice to induce type I diabetes mellitus. The insulin-positive cell population produced using (1) ALK5iII and the insulin-positive cell population produced using (4) a combination of the ALK5 inhibitor and the CDK8/19 inhibitor (SB/Sen), obtained above in Example 3 were each embedded in a fibrin gel obtained by mixing 10 mg/mL fibrinogen (Merck Millipore) with 50 IU/mL thrombin solution (Sigma-Aldrich Co., LLC), and subcutaneously transplanted to the mouse models mentioned above (3 × 10⁶ cells/mouse). The amounts of glucose and human C-peptide in plasma in each mouse model were measured every 2 or 4 weeks using ACCU-CHEK Aviva (Roche Diagnostics, Basel, Switzerland) and Ultrasensitive C-peptide ELISA (Mercodia AB, Uppsala, Sweden) according to the manufacturers' instructions.

The insulin-positive cell population produced using (1) ALK5iII and the insulin-positive cell population produced using (4) a combination of the ALK5 inhibitor and the CDK8/19 inhibitor (SB/Sen), transplanted in the mouse models exhibited an equivalent blood glucose-lowering effect and human C-peptide-secreting effect (Figures 7A and 7B), and all the mice given any of the transplants reached a normal blood glucose level within 8 weeks.

Each mouse model was subjected to an oral glucose load test on 21 weeks after transplantation. As a result, all the mice responded to glucose load in a manner peaking in 15 minutes (Figures 7C and 7D).

The insulin-positive cell population produced using (1) ALK5iII and the insulin-positive cell population produced using (4) a combination of the ALK5 inhibitor and the CDK8/19 inhibitor (SB/Sen), transplanted in the mouse models were excised after a lapse of 6 months from transplantation and immunostained using antibodies against various markers for insulin-positive cells. As a result, the appearance of glucagon-positive α cells in a larger amount than that at the time of transplantation was confirmed in both the cell populations.

The results described above demonstrated that a cell population is treated with a combination of the nonmutagenic ALK5 inhibitor and the CDK8/19 inhibitor instead of mutagenic ALK5iII in the process of inducing the differentiation of a pancreatic progenitor cell population into an insulin-positive cell population, whereby an insulin-positive cell population having *in vitro* and *in vivo* profiles equivalent to an insulin-positive cell population obtained by a conventional method using ALK5iII can be produced.

## Claims

1. A method for producing an insulin-positive cell population, comprising
culturing and differentiating a pancreatic progenitor cell population or a cell population at a later stage of differentiation in a medium containing a factor having ALK5-inhibiting activity and CDK8/19-inhibiting activity, wherein the medium contains substantially no 2-(3-(6-methylpyridin-2-yl)-1H-pyrazol-4-yl)-1,5-naphthyridine.

2. The method according to claim 1, wherein the factor having ALK5-inhibiting activity and CDK8/19-inhibiting activity comprises an ALK5 inhibitor and a CDK8/19 inhibitor.

3. The method according to claim 2, wherein the CDK8/19 inhibitor is one or more compounds selected from the group consisting of diethyl (E)-(4-(3-(5-(4-fluorophenyl)-1-methyl-1H-pyrazol-4-yl)acrylamido)benzyl)phosphonate, 2-(4-(4-(isoquinolin-4-yl)phenyl)-1H-pyrazol-1-yl)-N,N-dimethylacetamide, 4-((2-(6-(4-methylpiperazine-1-carbonyl)naphthalen-2-yl)ethyl)amino) quinazoline-6-carbonitrile, 4-(4-(2,3-dihydrobenzo[b][1,4]dioxin-6-yl)-1H-pyrazol-3-yl)benzene-1,3-diol, 3-(2-(imidazo[1,2-b]pyridazin-6-ylthio)ethyl)-4-(naphthalen-1-ylsulfornyl)-3,4-dihydroquinoxalin-2(1H)-one, and (E)-3-(4-(1-cyclopropyl-1H-pyrazol-4-yl)pyridin-3-yl)-N-(4-(morpholinomethyl)phenyl)acrylamide, or salt(s) thereof.

4. The method according to claim 2, wherein the ALK5 inhibitor is one or more compounds selected from the group consisting of 4-[4-(1,3-benzodioxol-5-yl)-5-(2-pyridinyl)-1H-imidazol-2-yl]-benzamide, 6-[2-tert-butyl-5-(6-methylpyridin-2-yl)-1H-imidazol-4-yl]quinoxaline, 3-[[5-(6-methyl-2-pyridinyl)-4-(6-quinoxalinyl)-1H-imidazol-2-yl]methyl]benzamide, and 2-fluoro-N-[[5-(6-methylpyridin-2-yl)-4-([1,2,4]triazolo[1,5-a]pyridin-6-yl)-1H-imidazol-2-yl]methyl]aniline, or salt(s) thereof.

5. The method according to claim 2, wherein the CDK8/19 inhibitor is 4-((2-(6-(4-methylpiperazine-1-carbonyl)naphthalen-2-yl)ethyl)amino)quinazoline-6-carbonitrile or 2-(4-(4-(isoquinolin-4-yl)phenyl)-1H-pyrazol-1-yl)-N,N-dimethylacetamide or a salt thereof, and the ALK5 inhibitor is 4-[4-(1,3-benzodioxol-5-yl)-5-(2-pyridinyl)-1H-imidazol-2-yl]-benzamide or a salt thereof.

6. The method according to claim 1 wherein the pancreatic progenitor cell population or the cell population at a later stage of differentiation is a cell population produced by the induction of differentiation from pluripotent stem cells.

7. A differentiation medium for a pancreatic progenitor cell population or a cell population at a later stage of differentiation, comprising a factor having ALK5-inhibiting activity and CDK8/19-inhibiting activity and containing substantially no 2-(3-(6-methylpyridin-2-yl)-1H-pyrazol-4-yl)-1,5-naphthyridine.

8. The medium according to claim 7, wherein the factor having ALK5-inhibiting activity and CDK8/19-inhibiting activity comprises an ALK5 inhibitor and a CDK8/19 inhibitor.

9. The medium according to claim 8, wherein the CDK8/19 inhibitor is one or more compounds selected from the group consisting of diethyl (E)-(4-(3-(5-(4-fluorophenyl)-1-methyl-1H-pyrazol-4-yl)acrylamido)benzyl)phosphonate, 2-(4-(4-(isoquinolin-4-yl)phenyl)-1H-pyrazol-1-yl)-N,N-dimethylacetamide, 4-((2-(6-(4-methylpiperazine-1-carbonyl)naphthalen-2-yl)ethyl)amino) quinazoline-6-carbonitrile, 4-(4-(2,3-dihydrobenzo[b][1,4]dioxin-6-yl)-1H-pyrazol-3-yl)benzene-1,3-diol, 3-(2-(imidazo[1,2-b]pyridazin-6-ylthio)ethyl)-4-(naphthalen-1-ylsulfornyl)-3,4-dihydroquinoxalin-2(1H)-one, and (E)-3-(4-(1-cyclopropyl-1H-pyrazol-4-yl)pyridin-3-yl)-N-(4-(morpholinomethyl)phenyl)acrylamide, or salt(s) thereof.

10. The medium according to claim 8, wherein the ALK5 inhibitor is one or more compounds selected from the group consisting of 4-[4-(1,3-benzodioxol-5-yl)-5-(2-pyridinyl)-1H-imidazol-2-yl]-benzamide, 6-[2-tert-butyl-5-(6-methylpyridin-2-yl)-1H-imidazol-4-yl]quinoxaline, 3-[[5-(6-methyl-2-pyridinyl)-4-(6-quinoxalinyl)-1H-imidazol-2-yl]methyl]benzamide, and 2-fluoro-N-[[5-(6-methylpyridin-2-yl)-4-([1,2,4]triazolo[1,5-a]pyridin-6-yl)-1H-imidazol-2-yl]methyl]aniline, or salt(s) thereof.

11. The medium according to claim 8, wherein the CDK8/19 inhibitor is 4-((2-(6-(4-methylpiperazine-1-carbonyl)naphthalen-2-yl)ethyl)amino)quinazoline-6-carbonitrile or 2-(4-(4-(isoquinolin-4-yl)phenyl)-1H-pyrazol-1-yl)-N,N-dimethylacetamide or a salt thereof, and the ALK5 inhibitor is 4-[4-(1,3-benzodioxol-5-yl)-5-(2-pyridinyl)-1H-imidazol-2-yl]-benzamide or a salt thereof.

12. The medium according to claim 7, wherein the pancreatic progenitor cell population or the cell population at a later stage of differentiation is a cell population produced by the induction of differentiation from pluripotent stem cells.
